# EUROPÄISCHE PATENTSCHRIFT

(11) **EP 3 548 866 B1**
(45) Veröffentlichungstag und Bekanntmachung des Hinweises auf die Patenterteilung: **17.06.2020**
(21) Anmeldenummer: 17793955.0
(22) Anmeldetag: 06.11.2017
(51) Int. Cl.: G01N 3/46

(54) **MESSVORRICHTUNG, MESSANORDNUNG UND VERFAHREN ZUR ERMITTLUNG VON MESSSIGNALEN WÄHREND EINER EINDRINGBEWEGUNG EINES EINDRINGKÖRPERS IN EINE OBERFLÄCHE EINES PRÜFKÖRPERS**
MEASURING SYSTEM, MEASURING ARRANGEMENT AND METHOD FOR DETERMINING MEASURING SIGNALS DURING A PENETRATION MOVEMENT OF A PENETRATION BODY INTO A SURFACE OF A TEST BODY
DISPOSITIF DE MESURE, SYSTÈME DE MESURE ET PROCÉDÉ POUR DÉTERMINER DES SIGNAUX DE MESURE PENDANT UN MOUVEMENT DE PÉNÉTRATION D'UN INDENTEUR DANS UNE SURFACE D'UN ÉCHANTILLON

(30) Priorität: 29.11.2016 DE 102016123010
(43) Veröffentlichungstag der Anmeldung: 09.10.2019
(73) Patentinhaber: Helmut Fischer GmbH Institut für Elektronik und Messtechnik, 71069 Sindelfingen (DE)
(72) Erfinder: FISCHER, Helmut, 6315 Oberägeri (CH)
(74) Vertreter: Mammel und Maser
(86) Internationale Anmeldenummer: PCT/EP2017/078275
(87) Internationale Veröffentlichungsnummer: WO 2018/099688

(56) Entgegenhaltungen:
- DE-A1- 4 343 612
- DE-A1-102014 202 839
- JP-A- S5 548 635
- US-A- 5 866 807

## Beschreibung

Die Erfindung betrifft eine Messvorrichtung sowie eine Messanordnung und ein Verfahren zur Erfassung von Messsignalen während einer Eindringbewegung eines Eindringkörpers in eine Oberfläche eines Prüfkörpers als auch zur Ermittlung der Kratzfestigkeit der Oberfläche des Prüfkörpers sowie zur Ermittlung der Oberflächenrauigkeit des Prüfkörpers.

Aus der JP S55 48635 A ist eine Messvorrichtung mit einem Eindringkörper bekannt, welcher eine Krafterzeugungseinrichtung aufweist. Diese Krafterzeugungseinrichtung umfasst eine fest am Gehäuse angeordnete Spule auf einem Magnetkern. Gegenüberliegend zum Magnetkern ist eine Übertragungseinrichtung vorgesehen, welche einen Dauermagneten umfasst. Der an der Übertragungseinrichtung angeordnete Dauermagnet und der fest am Gehäuse angeordnete magnetische Kern, der die Spule aufnimmt, weisen mit gleichen Polen aufeinander.

Aus der US 5,866,807 A ist eine Härtemessvorrichtung bekannt, bei welcher mit einem biegbaren Hebel an dessen einen Ende ein Eindringkörper vorhanden ist, der mit einer vorbestimmten Kraft eine Eindringbewegung des Eindringkörpers in die Oberfläche des Prüfkörpers angesteuert wird.

Aus der DE 699 17 780 T2 sind eine Messeinrichtung sowie ein Verfahren zur Messung der Kratzfestigkeit einer Oberfläche eines Prüfkörpers bekannt, welche einen Messtisch zur Aufnahme eines Prüfkörpers sowie eine Handhabungseinrichtung zum Überführen der Messvorrichtung aus einer Ausgangsposition in eine Messposition aufweist. Des Weiteren ist eine Steuerung vorgesehen, durch welche die Messvorrichtung nach dem Aufsetzen eines Prüfkörpers auf der zu prüfenden Oberfläche sowohl eine Verfahrbewegung des Messtisches entlang einer Achse als auch eine Eindringbewegung des Eindringkörpers angesteuert wird, so dass während der Verfahrbewegung des Messtisches der Eindringkörper in die Oberfläche des Prüfkörpers eindringt.

Zur Ansteuerung der Eindringbewegung des Eindringkörpers nach dem Aufsetzen auf der Oberfläche des Prüfkörpers weist die Messvorrichtung einen piezoelektrischen Aktuator auf, der eine erste Halteplatte beaufschlagt, welche mittels zwei Blattfederpaaren auf und ab bewegbar ist. Diese Halteplatte nimmt eine weitere Platte auf, welche wiederum durch zwei Blattfederpaare auf und ab bewegbar gelagert ist, wobei in dieser Platte der Eindringkörper angeordnet ist. Zwischen der Halteplatte und der den Eindringkörper aufnehmenden Platte ist eine Messvorrichtung vorgesehen, welche den Eindringweg misst. Des Weiteren ist daneben eine Messvorrichtung für die Ermittlung der Normalkraft angeordnet.

Diese Messvorrichtung weist den Nachteil auf, dass zwischen dem piezoelektrischen Aktuator und der Eindringspitze ein konstruktiv aufwändiger und schwerer Aufbau durch die Halteplatte als auch durch die den Eindringkörper aufnehmende Platte sowie durch die jeweils zur Lagerung ausgewählten Blattfederpaare vorgesehen ist. Dadurch ist nicht nur ein großer Bauraum erforderlich, sondern auch der piezoelektrische Antrieb entsprechend groß auszulegen, um die Kraft zur Ansteuerung einer Eindringbewegung aufzubringen. Darüber hinaus ist diese Messvorrichtung träge aufgrund des aufwändigen und konstruktiven Aufbaus. Weiterhin ist die Messvorrichtung aufgrund der Ansteuerung des Eindringkörpers mittels eines hochpräzisen piezoelektrischen Aktuators kostenintensiv.

Der Erfindung liegt die Aufgabe zugrunde, eine Messvorrichtung zur Erfassung von Messsignalen während einer Eindringbewegung eines Eindringkörpers in eine Oberfläche eines Prüfkörpers, insbesondere zur Ermittlung der Kratzfestigkeit der Oberfläche des Prüfkörpers oder zur Erfassung von Messsignalen während einer Abtastbewegung eines Eindringkörpers auf der Oberfläche des Prüfkörpers, insbesondere zur Ermittlung der Oberflächenrauigkeit des Prüfkörpers, sowie eine Messanordnung als auch ein Verfahren zur Ermittlung von Messsignalen während einer Eindringbewegung eines Eindringkörpers, insbesondere zur Ermittlung der Kratzfestigkeit der Oberfläche des Prüfkörpers oder während einer Abtastbewegung des Eindringkörpers, zu schaffen, wodurch eine erhöhte Messgenauigkeit und eine Kostenreduzierung ermöglicht wird.

Die der Erfindung zugrundeliegende Aufgabe wird durch eine Messvorrichtung mit einem Eindringkörper und einer Krafterzeugungseinrichtung, mit welcher der Eindringkörper wirkverbunden ist, gelöst, bei der der Eindringkörper in eine zu messende Oberfläche des Prüfkörpers eindringt oder eine zu messende Oberfläche des Prüfkörpers abtastet. Zur Messung der Eindringtiefe oder der Oberflächenrauigkeit ist zumindest eine Messeinrichtung vorgesehen. Die Eindringbewegung des Eindringkörpers oder die Abtastbewegung des Eindringkörpers ist durch die Krafterzeugungseinrichtung mittels einer Magnetkraft ansteuerbar. Der Einsatz einer solchen Krafterzeugungseinrichtung, welche eine Antriebseinrichtung und eine magnetische Übertragungseinrichtung umfasst, bei welcher mittels einer Magnetkraft die Eindringbewegung oder die Abtastbewegung des Eindringkörpers angesteuert ist, weist den Vorteil auf, dass eine körperliche Entkopplung der Antriebseinrichtung und des Eindringkörpers gegeben ist. Dadurch ist eine reibungsfreie Kraftübertragung vom Antrieb auf den Eindringkörper ermöglicht. Zudem wird eine Änderung in der Magnetkraft unmittelbar in eine Eindringbewegung des Eindringkörpers in die Oberfläche des Prüfkörpers oder in eine Auflagekraft für eine Abtastbewegung des Eindringkörpers umgesetzt. Die Zunahme der Magnetkraft bedeutet somit eine unmittelbare Zunahme der Kraft für den Eindringkörper und umgekehrt. Durch die Ausgestaltung der Krafterzeugungseinrichtung mit einer Magnetkraft ist somit eine hysteresefreie Ansteuerung der Eindringbewegung des Eindringkörpers ermöglicht. Zudem können durch die Krafterzeugungseinrichtung Temperatureinflüsse ausgeschlossen werden. Darüber hinaus sind auch weitere nachteilige Einflüsse ausgeschlossen, da die Magnetkraft nicht komprimierbar ist. Durch die hysteresefrei ansteuerbare Kraftübertragung kann eine exakte Einstellung und/oder hohe Wiederholbarkeit einer Eindringkraft oder Auflagekraft für eine Abtastbewegung des Eindringkörpers einstellbar sein. Zudem kann eine massereduzierte Anordnung einer Krafterzeugungseinrichtung erzielt werden. Auch weist eine solche Krafterzeugungseinrichtung den Vorteil auf, dass eine einmal eingestellte Magnetkraft konstant gehalten werden kann.

Die magnetische Übertragungseinrichtung der Messvorrichtung weist einen ersten und einen zweiten Magnetpol auf, die mit Abstand einander gegenüberliegend angeordnet sind, wobei die Magnetpole mit gleichen Polen zueinander ausgerichtet sind. Bevorzugt sind Permanentmagnete vorgesehen. Der erste und zweite Magnetpol können durch einen oder mehrere Permanentmagnete ausgebildet sein, die gemeinsam oder auch zueinander beabstandet ausgerichtet sein können. Beispielsweise können zwei zueinander beabstandete oder beispielsweise drei auf einem Kreis angeordnete Permanentmagnete einen ersten und/oder zweiten Magnetpol bilden. Dadurch kann zwischen dem ersten und zweiten Magnetpol eine abstoßende Kraft erzeugt werden. Da die jeweiligen Magnetfelder der Magnetpole nicht komprimierbar sind, kann mit zunehmender Verringerung des Abstandes eine definierte Kraftzunahme erzeugt und erzielt werden, die für eine Verfahrbewegung des Eindringkörpers vorgesehen ist.

Der zweite Magnetpol ist am Übertragungselement vorgesehen, welches an seinem gegenüberliegenden Ende den Eindringkörper aufnimmt, wobei das Übertragungselement längs einer Verfahrachse verfahrbar im Gehäuse geführt ist. Die Verfahrachse ist vorzugsweise senkrecht zu einer Grundplatte des Gehäuses ausgerichtet beziehungsweise die Verfahrachse liegt bevorzugt in der Achse der Eindringbewegung des Eindringkörpers. Dabei kann eine längs des Antriebselements wirkende Magnetkraft in eine Verfahrbewegung entlang der Längsachse des Übertragungselements umgesetzt werden. Dies ermöglicht eine verlustfreie Anordnung und Kraftübertragung.

Der erste Magnetpol der magnetischen Übertragungseinrichtung ist mit der Antriebseinrichtung verbunden, durch welche eine Verfahrbewegung des ersten Magnetpols entlang einer Verfahrachse angesteuert wird. Diese Verfahrachse kann in der Achse der Eindringbewegung des Eindringkörpers oder parallel dazu liegen. Dadurch wird eine Verfahrbewegung des ersten Magnetpols unmittelbar auf den zweiten Magnetpol zu angesteuert, wobei insbesondere bei einer Deckungsgleichheit der Verfahrachsen eine besonders günstige, insbesondere verkippungsfreie, Kraftübertragung der Magnetkraft vom ersten Magnetpol auf den zweiten Magnetpol gegeben ist. Alternativ kann die Verfahrachse des ersten Magnetpols senkrecht zur Achse der Eindringbewegung des Eindringkörpers ausgerichtet sein. Üblicherweise ist die Verfahrachse des Eindringkörpers senkrecht zur Oberfläche des Messgegenstandes ausgerichtet und liegt somit bevorzugt in der Vertikalen. Bei der alternativen Ausführungsform liegt die Verfahrachse des ersten Magnetpols somit in der Horizontalen. Eine solche Anordnung weist den Vorteil auf, dass eine flachbauende Messvorrichtung geschaffen werden kann.

Bevorzugt ist vorgesehen, dass durch eine Verfahrbewegung des ersten Magnetpols in Richtung auf den zweiten Magnetpol die Verfahrbewegung des Eindringkörpers in Richtung auf den Prüfkörper und eine Eindringkraft in den Prüfkörper oder eine Auflagekraft zum Abtasten der Oberfläche des Prüfkörpers einstellbar ist. Dadurch sind einfache Verhältnisse gegeben, wobei lediglich durch eine Zustellbewegung des ersten Magnetpols die Ansteuerung des Eindringkörpers über den zweiten Magnetpol ermöglicht wird.

Vorteilhafterweise ist vorgesehen, dass das Übertragungselement stift- oder rohrförmig ausgebildet ist. Dadurch kann eine steife Ausgestaltung des Übertragungselementes als auch eine leichtbauende Ausführungsform geschaffen sein.

Das Übertragungselement ist bevorzugt durch eine Führung verfahrbar aufgenommen, welche an einer Haltevorrichtung im Gehäuse befestigt ist. Diese Führung weist bevorzugt zumindest zwei zueinander beabstandete und nachgiebige Elemente auf, insbesondere zwei parallel zueinander beabstandete Blattfederelemente oder zwei parallel zueinander beabstandete Druckmembranelemente, die in der Verfahrachse der Antriebseinrichtung verfahrbar geführt oder entlang der Verfahrachse auslenkbar sind. Die Blattfederelemente oder Druckmembranelemente greifen einerseits am Übertragungselement an und sind mit deren gegenüberliegenden Ende an einer Haltevorrichtung des Gehäuses gehalten.

Die Führung wird bevorzugt aus nicht-magnetisierbaren Materialien hergestellt.

Gemäß einer ersten Ausführungsform kann vorgesehen sein, dass die Blattfederelemente oder Druckmembranelemente der Haltevorrichtung fest eingespannt gehalten sind. Dies ermöglicht, dass in die Blattfederelemente oder Druckmembranelemente an die jeweilige Aufgabe und/oder Größe der Messvorrichtung in einfacher Weise durch Austausch anpassbar sind. Zudem kann ein modularer Aufbau geschaffen werden.

Alternativ kann vorgesehen sein, dass die Haltevorrichtung und Blattfederelemente einstückig ausgebildet sind und vorzugsweise die Blattfederelemente durch Erodieren oder Feinstbearbeitung hergestellt sind. Eine solche Anordnung ermöglicht einen kompakten Aufbau, bei welchem gleichzeitig eine Verfahrbewegung der Blattfederelemente durch ein zwischen dem Blattfederelement erstreckender Zwischenkörper der Haltevorrichtung begrenzt ist.

Die Blattfederelemente weisen bevorzugt einen der Haltevorrichtung zugeordneten Klemmbereich und gegenüberliegend eine an dem Übertragungsstift angreifenden Aufnahmebereich sowie dazwischenliegend einen Distanzabschnitt auf, wobei der Klemmbereich und der Distanzabschnitt sowie der Aufnahmebereich und der Distanzabschnitt jeweils mittels Festkörpergelenken miteinander verbunden sind. Diese Anordnung weist den Vorteil auf, dass der Klemmbereich und der Aufnahmebereich bei einer Auslenkung des Blattfederelementes im Wesentlichen parallel ausgerichtet bleiben können. Vorteilhafterweise ist vorgesehen, dass die Festkörpergelenke als Scharniere ausgebildet sind, die in eine Raumrichtung nachgiebig und in die beiden weiteren Raumrichtungen steif sind. Diese können in der Dicke gegenüber dem Klemmbereich, dem Aufnahmebereich und dem Distanzabschnitt reduziert sein. Durch die Dicke der Festkörpergelenke kann die aufzubringende Kraft für eine Auslenkung der Blattfederelemente bestimmt sein.

Eine vorteilhafte Ausgestaltung der Festkörpergelenke in den Blattfederelementen sieht vor, dass sich diese über die gesamte Breite des Blattfederelementes erstrecken und vorzugsweise zumindest eine Langlochausnehmung aufweisen. Durch eine solche Langlochausnehmung kann wiederum die Kraft für eine Auslenkbewegung in einfacher Weise anpassbar sein und reduziert werden.

Das Gehäuse der Messvorrichtung weist bevorzugt eine Grundplatte mit einer Ausnehmung auf, entlang deren Längsachse die Verfahrbewegung des Eindringkörpers ausgerichtet ist, wobei der Eindringkörper durch die Öffnung hindurchführbar ist und in einer nach außen gegenüber der Grundplatte hervorstehende Positionen positionierbar ist. Somit können eine Antriebsachse der Antriebseinrichtung zur Ansteuerung der Verfahrbewegung des Eindringkörpers sowie eine Längsachse des Übertragungselements, welches den Eindringkörper aufnimmt, in einer gemeinsamen Achse liegen. Dies ermöglicht eine unmittelbare und direkte Ansteuerung einer Verfahrbewegung des Eindringkörpers.

Bevorzugt ist vorgesehen, dass die Führung das Übertragungselement und den daran angeordneten Eindringkörper in einer Ausgangsposition hält, in der der Eindringkörper gegenüber einer Unterseite des Gehäuses, welche zum Prüfkörper ausgerichtet ist, nach innen zurückversetzt angeordnet ist. Dies weist den Vorteil auf, dass ein Schutz gegen Beschädigungen des Eindringkörpers gegeben ist. In dieser Ausgangsposition ist bevorzugt das Übertragungselement mit dem daran vorgesehenen Magnetpol im Aufnahmeelement und dem gegenüberliegend angeordneten Eindringkörper im Gleichgewicht gehalten. Der Eindringkörper ist bevorzugt innerhalb einer Öffnung in der Grundplatte des Gehäuses positioniert. In dieser Öffnung kann des Weiteren auch ein Aufsetzring positioniert sein, welcher eine Durchgangsbohrung aufweist. Der Aufsetzring kann gegenüber der Unterseite des Gehäuses hervorstehen, jedoch ist der Eindringkörper in der Ausgangsposition auch gegenüber einer Aufsetzfläche des Aufsetzringes bevorzugt nach innen zurückversetzt.

Benachbart zur Ausnehmung ist in der Grundplatte des Gehäuses bevorzugt eine erste Messeinrichtung, insbesondere ein Abstandssensor, vorgesehen, welche einen Messfühler aufweist, der einem innenliegenden Ende des Eindringkörpers zugeordnet ist. Dadurch kann während einer Verfahrbewegung oder einer Eindringbewegung des Eindringkörpers in den Prüfkörper die tatsächliche Verfahrbewegung erfasst und über die Auswertung der Härte des Prüfkörpers oder eines Kratzers oder auch für die Ermittlung der Oberflächenrauigkeit erfasst und an eine Steuereinrichtung weitergeleitet werden.

Die Antriebseinrichtung der Krafterzeugungseinrichtung ist vorteilhafterweise an einem der Grundplatte gegenüberliegend vorgesehenen Deckelelement des Gehäuses vorgesehen, welches zumindest ein längsverstellbares Antriebselement aufweist, das vorzugsweise in einer Verfahrachse der Verfahrbewegung des Eindringkörpers liegt. Dies weist den Vorteil auf, dass die Einleitung einer Kraft zur Erzeugung einer Verfahrbewegung in der Verfahrachse des Eindringkörpers liegt, so dass Verluste minimiert werden können.

Das Antriebselement nimmt an einem zum Übertragungsstift weisenden Ende den ersten Magnetpol auf. Dies weist den Vorteil auf, dass durch eine definierte Verfahrbewegung des Antriebselementes, welches dem Deckelelement zugeordnet ist, eine gezielte Abstandsänderung zum gegenüberliegenden beziehungsweise zweiten Magnetpol einstellbar ist, das heißt, das mit zunehmender Verringerung des Abstandes eine Erhöhung der Magnetkraft einhergeht.

Das Antriebselement ist beispielsweise als eine Antriebsspindel ausgebildet und bevorzugt mit einer Führung verdrehsicher geführt, wobei diese Führung insbesondere am Deckelelement vorgesehen ist. Alternativ kann das Antriebselement als Teleskopspindel ausgebildet sein. Die Verfahrbewegung des Antriebselementes wird mit einem Drehantrieb und mit einem Antriebsmotor angesteuert. Bevorzugt ist ein elektrischer Antriebsmotor vorgesehen, der einen Drehantrieb antreibt, um eine definierte Zustellbewegung des Antriebselementes zu erzielen und vorzugsweise die Zustellbewegung zu dekodieren, um den Verfahrweg exakt bestimmen zu können.

Alternativ zum elektrischen Antrieb kann auch ein pneumatischer, hydraulischer oder elektromagnetischer Antrieb vorgesehen sein.

Vorteilhafterweise ist der Drehantrieb als Zahnriemenantrieb ausgebildet und treibt das Antriebselement an, das durch die Führung verdrehgesichert ist. Dadurch ist eine konstruktiv einfache Ausgestaltung gegeben. Durch die Steigung eines Gewindes an der Antriebsspindel oder der Gewinde von der Teleskopspindel ist eine definierte Zunahme einer Verfahrbewegung in Abhängigkeit der Umdrehung einstellbar.

Eine weitere alternative Ausgestaltung der Messvorrichtung sieht vor, dass die Verfahrachse des Antriebselements senkrecht zur Verfahrachse des Eindringkörpers ausgerichtet ist und das Antriebselement eine Verfahrbewegung des zumindest einen ersten Magnetpols entlang der Verfahrachse senkrecht zur Verfahrachse des Eindringkörpers ansteuert, bis dieser erste Magnetpol in eine Position mit einer teilweisen Überdeckung oder in eine deckungsgleiche Position zum zweiten Magnetpol übergeführt ist. Alternativ kann vorgesehen sein, dass der erste Magnetpol durch zwei oder mehrere Permanentmagnete gebildet ist, welche gleichzeitig in eine Position mit einer teilweisen Überdeckung oder in eine deckungsgleiche Position zu einer entsprechenden Anzahl von Permanentmagneten überführbar ist, die den zweiten Magnetpol bilden. Sofern beispielsweise zwei Permanentmagnete einen ersten Magnetpol bilden, ist der zweite Magnetpol ebenfalls durch zwei Permanentmagnete ausgebildet. Durch eine gleichzeitige Verfahrbewegung der den ersten Magnetpol bildenden Permanentmagnete von einem Bereich außerhalb dem Magnetfeld der den zweiten Magnetpol bildenden Permanentmagnete in eine Position mit einer teilweisen Überdeckung oder in eine deckungsgleiche Anordnung wird eine gleichmäßige Einwirkung des Kraftfeldes auf die zwei Permanentmagnete des zweiten Magnetpols erzielt. Dadurch kann eine verkippungsfreie Ansteuerung einer Verfahrbewegung des Übertragungselementes entlang der Verfahrachse des Eindringkörpers erzielt wird.

Das Antriebselement für die vorbeschriebene alternative Ausgestaltung der Messvorrichtung umfasst bevorzugt ein Paar einander zugeordnete Antriebselemente, insbesondere Zahnstangen, welche mit einem Drehantrieb senkrecht zur Verfahrachse des Eindringkörpers ansteuerbar sind und vorzugsweise entlang von Führungsschienen verfahrbar sind. Diese Führungsschienen sind senkrecht zur Verfahrbewegung des Eindringkörpers, insbesondere senkrecht zur Verfahrachse des Übertragungselementes, ausgerichtet. An jedem Antriebselement, insbesondere jeder Zahnstange, ist ein Permanentmagnet vorgesehen, die gemeinsam einen ersten Magnetpol bilden. Durch eine solche Anordnung können beide Antriebselemente, insbesondere Zahnstangen, mit einem Antriebsrad angesteuert werden, wodurch eine synchrone Verfahrbewegung der Permanentmagnete des ersten Magnetpols zur teilweisen Überdeckung oder zur deckungsgleichen Anordnung der Permanentmagnete des zweiten Magnetpols angesteuert werden. Vorteilhafterweise kann eine Antriebsachse des Antriebsrades der beiden Antriebselemente geringfügig außerhalb eines Winkels von 90° zur Verfahrrichtung der Antriebselemente ausgerichtet sein. Dadurch kann eine spielfreie Einstellung erzielt werden.

Des Weiteren ist bei der alternativen Ausführungsform bevorzugt vorgesehen, dass an dem Übertragungselement eine Aufnahmevorrichtung vorgesehen ist, welche zumindest einen in der Verfahrachse angeordneten Permanentmagneten oder zwei oder mehrere Permanentmagnete im gleichen Abstand zur Verfahrachse des Übertragungselementes zur Bildung des zweiten Magnetpols aufnimmt. Dadurch ist hinreichend Raum gegeben, um beispielsweise zwei gegenläufig zueinander verfahrbare Permanentmagnete des ersten Magnetpols auf die Permanentmagnete des zweiten Magnetpols zuzubewegen und in eine deckungsgleiche Anordnung zur maximalen Kraftübertragung zu positionieren.

Vorteilhafterweise ist die Antriebsbewegung des Antriebselementes mit einer dritten Messeinrichtung, insbesondere einem Drehgeber, überwacht. Durch die Kenntnis des magnetischen Flusses der Magnetpole kann mit zunehmender Verringerung des Abstands zwischen den Magnetpolen die zunehmende Kraft ermittelt werden. Durch diesen Drehgeber kann zur Änderung des Abstandes der Magnetpole und somit die aufgebrachte Kraft auf den Eindringkörper exakt erfasst werden, so dass durch die Steuerungseinrichtung aufgrund des Verfahrweges des Antriebselementes die einwirkende Kraft auf die Oberfläche des Prüfkörpers als Auswertegröße zugrundegelegt werden kann.

Eine weitere bevorzugte Ausgestaltung der Messvorrichtung sieht vor, dass zwischen dem Antriebselement und dem daran angeordneten Magnetpol eine vierte Messeinrichtung, insbesondere ein Kraftsensor, vorgesehen ist. Dadurch kann eine zusätzliche Überwachung der einwirkenden Kraft durch den Magnetpol des Antriebselements auf den gegenüberliegenden Magnetpol am Übertragungsstift erfasst und/oder überwacht werden.

Eine weitere bevorzugte Ausgestaltung sieht vor, dass dem am Übertragungselement angeordneten Magnetpol eine Schwingungsdämpfungseinrichtung zugeordnet ist. Dadurch können unerwünschte Abhebebewegungen des Eindringkörpers während einer Messung mit dieser Messvorrichtung reduziert oder verhindert werden. Insbesondere ist dies bei der Ermittlung der Kratzfestigkeit einer Oberfläche des Eindringkörpers von Vorteil.

Die Schwingungsdämpfungseinrichtung ist bevorzugt gemäß einer ersten Ausführungsform durch ein aus einem ferromagnetischen Material hergestellten Umhausung, insbesondere Rohr, ausgebildet, welches den am Übertragungselement angeordneten zweiten Magnetpol umgibt, wobei der Magnetpol in einer Ausgangsposition des Eindringkörpers zumindest teilweise in die Schwingungsdämpfungseinrichtung eingetaucht ist. Mit zunehmender Verfahrbewegung des Eindringkörpers auf den Prüfkörper zu wird der zweite Magnetpol gegenüber der Schwingungsdämpfungseinrichtung geringfügig herausbewegt, das heißt, dass bei einer möglichen Abhebebewegung des Eindringkörpers vom Prüfkörper ein Eintauchen des Magnetpols in die Schwingungsdämpfungseinrichtung erfolgt, welche bewirkt, dass die Magnetkraft verstärkt und somit der Eintauchbewegung entgegengewirkt wird.

Gemäß einer weiteren bevorzugten Ausführungsform der Messvorrichtung ist vorgesehen, dass zwischen den parallel zueinander beabstandeten Blattfederelementen ein Ausgleichselement vorgesehen ist, welches an der Haltevorrichtung gelagert ist und mit einem Ende in das Übertragungselement ragt, an welchem ein weiteres Blattfederelement vorgesehen ist, welches sich in Richtung auf ein den Magnetpol aufnehmendes Ende des Übertragungsstiftes erstreckt und daran befestigt ist. Durch dieses Blattfederelement kann eine Auslenkbewegung des Übertragungselementes bei einer Verfahrbewegung des Prüfkörpers relativ zum Eindringkörper bei der Ermittlung der Kratzfestigkeit entgegengewirkt werden kann. Zudem kann durch diese Anordnung auch eine Ausrichtung des Übertragungselementes in einer Grundposition oder Ausgangsposition innerhalb des Gehäuses der Messvorrichtung erzielt werden.

Eine bevorzugte Ausführungsform des Ausgleichselementes sieht vor, dass dieses mittels einer Spannbandlagerung an der Haltevorrichtung gelagert ist. Dadurch kann die Ausnivellierung des Übertragungsstiftes in einer Grund- oder Ausgangsposition erzielt werden.

Eine weitere bevorzugte Ausgestaltung der Messvorrichtung sieht vor, dass das entfernt zur Grundplatte angeordnete Blattfederelement oder Druckmembranelement in der Haltevorrichtung fest eingespannt ist und das nahe der Grundplatte angeordnete Blattfederelement oder Druckmembranelement bezüglich eines durch Längsschlitze gebildeten Abschnitts in einer Richtung senkrecht zur Verfahrachse des Übertragungselements verschiebbar gelagert ist. Vorzugsweise ist eine zweite Messeinrichtung zur Erfassung einer Verschiebebewegung des unteren Blattfederelements oder Abschnitts des Druckmembranelements vorgesehen. Diese zweite Messeinrichtung umfasst ein Sensorelement, welches eine Verschiebebewegung des unteren Blattfederelementes oder Abschnitts des Druckmembranelements während einer Verfahrbewegung des Übertragungselementes entlang dessen Längsachse oder der Verfahrachse des Antriebselements erfasst. Ebenfalls kann eine Auslenkung des Übertragungselementes beim Eindringen des Eindringkörpers in die Oberfläche des Prüfkörpers während der Durchführung einer Kratzfestigkeit ermittelt werden.

Die der Erfindung zugrunde liegende Aufgabe wird des Weiteren durch eine Messanordnung zum Erfassen von Messsignalen während einer Verfahrbewegung, insbesondere einer Eindringtiefe oder einer Abtastbewegung, eines Eindringkörpers in eine Oberfläche oder auf einer Oberfläche eines Prüfkörpers gelöst, bei der auf einem Grundkörper oder einer Basisplatte ein Messtisch zur Aufnahme des Prüfkörpers vorgesehen ist sowie eine Handhabungseinrichtung, insbesondere ein Stativ, welche eine Messvorrichtung aufnimmt, die über die Handhabungseinrichtung in eine Position zum Aufsetzen eines Eindringkörpers auf den Prüfkörper übergeführt wird, wobei die Verfahrbewegung zum Eindringen des Eindringkörpers in die Oberfläche des Prüfkörpers oder die Verfahrbewegung zum Abtasten der Oberfläche des Eindringkörpers durch eine Messvorrichtung nach einem oder mehreren der vorher beschriebenen Merkmale der Ausführungsformen angesteuert ist und durchgeführt wird.

Des Weiteren nimmt die Messanordnung bevorzugt eine optische Erfassungseinrichtung benachbart zur Messvorrichtung auf, welche die Eindringstelle, die Oberflächenrauheit oder bei Durchführung der Kratzfestigkeitsprüfung den eingebrachten Kratzer optisch erfasst und auswertet. Dabei ist bevorzugt der Messtisch zwischen der Messvorrichtung und der optischen Erfassungseinrichtung verfahrbar. Alternativ können die Messvorrichtung und die optische Erfassungseinrichtung zum Messtisch verfahrbar sein.

Des Weiteren ist bevorzugt eine Verfahrbewegung des Messtisches, insbesondere eine entlang einer Verfahrrichtung in der Ebene der Oberfläche des Prüfkörpers liegende Achse, durch die Steuerung angesteuert. Durch diese Steuerung kann somit beim Aufsetzen des Eindringkörpers auf die Oberfläche des Prüfkörpers, welcher eine Startposition bildet, und einer darauffolgenden gesteuerten Verfahrbewegung eine Oberflächenkontur oder eine Rauigkeit der Oberfläche erfasst werden. Dies kann auch für einen Pre-Scan einer Kratzfestigkeitsermittlung durchgeführt werden. Ebenso kann ausgehend von der Startposition während der Verfahrbewegung des Messtisches zum Eindringkörper eine Eindringbewegung des Eindringkörpers angesteuert werden, um einen Kratzer zu bilden. Auch kann ausgehend von der Startposition ein Post-Scan für eine Kratzfestigkeitsprüfung angesteuert werden.

Die der Erfindung zugrunde liegende Aufgabe wird des Weiteren durch ein Verfahren zum Erfassen von Messsignalen während einer Eindringbewegung eines Eindringkörpers in eine Oberfläche eines Prüfkörpers mit einer Messvorrichtung oder während einer Abtastbewegung eines Eindringkörpers auf einer Oberfläche eines Prüfkörpers, bei dem der Prüfkörper auf einem Messtisch positioniert und die Messvorrichtung auf den Prüfkörpers aufgesetzt wird, mit den Merkmalen des Anspruchs 14 gelöst.

Die Rückstellkraft der Blattfederelemente kann dabei vernachlässigt beziehungsweise in Abhängigkeit des Verfahrweges des Eindringkörpers entlang dessen Verfahrachse ermittelt und berücksichtigt werden. Die Ausgangskraft, mit welcher die Krafterzeugungseinrichtung während der Zustellbewegung der Messvorrichtung bis zum Aufsetzen auf den Prüfkörper auf den Eindringkörper wirkt, kann beispielsweise ein Kräftegleichgewicht zwischen den Rückstellkräften der Federelemente einerseits und der durch die Magnetkraft erzeugten Verfahrbewegung in Richtung des Eindringkörpers andererseits gebildet sein.

Bevorzugt ist ein erster Verfahrensschritt für eine Härtemessung an einer Oberfläche des Prüfkörpers vorgesehen, bei dem die Messvorrichtung auf den Prüfkörper zubewegt und beim Aufsetzen des Gehäuses der Messvorrichtung die Zustellbewegung stillgesetzt wird, wobei darauffolgend eine Verfahrbewegung des Eindringkörpers angesteuert wird, welcher in einer Ausgangslage gegenüber einer Außenseite der Messvorrichtung nach innen zurückversetzt ist, bis dieser auf dem Prüfkörper aufliegt, wobei diese Position als Nullposition für die nachfolgende Härtemessung an die Steuerung weitergeleitet wird. Dadurch kann eine definierte Ausgangslage für eine Messung erzielt werden. Zudem wird der Eindringkörper aus einer geschützten Ausgangsposition in eine Messposition übergeführt. Die Erfassung der Nullposition des Eindringkörpers, bei der diese auf der Oberfläche des Prüfkörpers aufliegt, wird vorteilhafterweise durch eine erste Messeinrichtung erfasst, die erkennt, dass keine Wegänderung gegeben ist, so dass dadurch ein Signal an die Steuerung weitergeleitet wird, um die Zustellbewegung der Messvorrichtung auf dem Prüfkörper stillzusetzen.

Des Weiteren ist bevorzugt ein erster Verfahrensschritt für eine Kraftfestigkeitsmessung vorgesehen, bei welcher vor dem Aufsetzen des Eindringkörpers auf die Oberfläche des Prüfkörpers dieser mit einer Zustellkraft beaufschlagt wird, so dass der Eindringkörper gegenüber einer Unterseite des Gehäuses frei nach außen hervorsteht. Darauffolgend wird die Messvorrichtung auf den Prüfkörper zubewegt, und beim Aufsetzen des Eindringkörpers auf den Prüfkörper wird die Verfahrbewegung der Messvorrichtung stillgesetzt. Bevorzugt wird diese Position als Nullposition wiederum an die Steuerung weitergeleitet für die nachfolgende Messung der Kratzfestigkeit.

Eine weitere bevorzugte Ausführungsform sieht vor, dass ausgehend von der Nullposition des Eindringkörpers die Krafterzeugungseinrichtung mit einer Prüfkraft beaufschlagt wird und eine Eindringbewegung des Eindringkörpers in die Oberfläche des Prüfkörpers mit einer ersten Messeinrichtung erfasst wird. Durch die Wegänderung während der Eindringbewegung als auch die Kenntnis der aufgebrachten Prüfkraft kann die Härte der Oberfläche des Prüfkörpers ermittelt werden. Gleichzeitig können diese Messergebnisse auch bei der Kratzfestigkeitsmessung einfließen.

Durch eine Zustellbewegung des Antriebselementes der Antriebseinrichtung wird eine Eindringbewegung des Eindringkörpers angesteuert, und eine Kraftübertragung von dem Antriebselement auf das magnetische Übertragungselement beziehungsweise den Eindringkörper durch eine magnetische Übertragungseinrichtung erfolgt.

Des Weiteren ist bevorzugt vorgesehen, dass die auf den Eindringkörper wirkende Kraft aus der Zustellbewegung des Antriebselementes, welche durch die dritte Messeinrichtung erfasst wird, errechnet oder durch eine vierte Messeinrichtung erfasst wird und dass durch die erste Messeinrichtung die Eindringtiefe des Eindringkörpers in den Prüfkörper erfasst wird und dass aus der errechneten oder der erfassten Eindringkraft durch die dritte oder vierte Messeinrichtung und der erfassten Eindringtiefe durch die erste Messeinrichtung in Abhängigkeit der Geometrie des Eindringkörpers zumindest die Härte der Oberfläche des Prüfkörpers bestimmt wird. Die vierte Messeinrichtung ist bevorzugt zwischen dem Antriebselement der Antriebsvorrichtung und dem vom Antriebselement vorgesehenen Magnetpol vorgesehen.

Zur Ermittlung einer Kratzfestigkeit der Oberfläche des Prüfkörpers wird bevorzugt während der Eindringbewegung des Eindringkörpers der Messtisch mit dem darauf aufgebrachten Prüfkörper in einer Richtung senkrecht zur Eindringbewegung des Eindringkörpers verfahren und ein Kratzer in die Oberfläche des Prüfkörpers eingebracht. Durch eine erste Messeinrichtung werden Messsignale bezüglich der Eindringtiefe in Abhängigkeit der Zeit und des Verfahrweges erfasst. Des Weiteren wird mittels einer zweiten Messeinrichtung der Messvorrichtung eine Auslenkung des Eindringkörpers entgegen der Verfahrrichtung des Messtisches erfasst. Des Weiteren wird eine auf den Eindringkörper wirkende Messkraft als Messsignal an die Steuerungseinrichtung weitergeleitet. Diese Messkraft kann aus der Zustellbewegung des Antriebselementes und der aus der magnetischen Übertragungseinrichtung resultierenden Zustellkraft des Eindringkörpers in die Oberfläche ermittelt werden. Alternativ kann für diese Ermittlung der Messkraft vorgesehen sein, dass mittels einer vierten Messeinrichtung Messsignale erfasst werden, wobei diese vierte Messeinrichtung zwischen einem Magnetpol der magnetischen Übertragungseinrichtung und dem Antriebselement positioniert ist, welche diesen Magnetpol aufnimmt. Aus diesen erfassten Signalen kann die Kratzfestigkeit einer Oberfläche des Prüfkörpers bestimmt werden.

Des Weiteren wird bevorzugt während dem Einbringen eines Kratzers in die Oberfläche des Prüfkörpers zusätzlich eine rechtwinklig zur Verfahrbewegung des Messtisches ausgerichtete Auslenkung des Eindringkörpers durch eine weitere Messvorrichtung erfasst. Dadurch kann zusätzlich eine Auswertung bezüglich der Oberfläche des Prüfkörpers geschaffen und insbesondere eine Aussage über die Homogenität des Materials erzielt werden.

Des Weiteren wird bevorzugt vor dem Einbringen eines Kratzers in den Prüfkörper die Messvorrichtung auf der Oberfläche aufgesetzt, in einer Richtung senkrecht zur Aufsetzbewegung des Prüfkörpers verfahren und die Oberfläche abgetastet. Dabei werden durch die erste Messvorrichtung Signale erfasst und als Vorkratz-Profil gespeichert. Durch einen sogenannten Pre-Scan kann der Verlauf der Oberfläche des Prüfkörpers bestimmt werden, so dass dieser weitere Parameter bei der anschließenden Bestimmung der Kratzfestigkeit mit einfließen kann.

Des Weiteren ist zur Ermittlung der Kratzfestigkeit vorgesehen, einen sogenannten Post-Scan durchzuführen. Hierzu wird bevorzugt nach dem Einbringen des Kratzers in den Prüfkörper die Messvorrichtung auf den Kratzer aufgesetzt und der Eindringkörper mit der Messvorrichtung in einer Richtung senkrecht zur Eindringbewegung des Prüfkörpers verfahren, also in dem Kratzer entlang geführt und die erfassten Messsignale abgespeichert.

Eine weitere bevorzugte Ausgestaltung des Verfahrens sieht vor, dass der Prüfdruck in der Krafterzeugungseinrichtung während der Abtastbewegung des Eindringkörpers konstant gehalten wird. Dabei kann der Eindringkörper unter gleichbleibenden Bedingungen entlang der Oberfläche des Prüfkörpers geführt werden, wobei die magnetische Übertragungseinrichtung dann quasi als ein steifes Stellglied ausgebildet ist, so dass die auf den Eindringkörper wirkende Verfahrbewegung längs dessen Längsachse aufgrund der Oberflächenrauheit unmittelbar übertragen und von zumindest der ersten Messeinrichtung erfasst werden kann.

Die Erfindung sowie weitere vorteilhafte Ausführungsformen und Weiterbildungen derselben werden im Folgenden anhand der in den Zeichnungen dargestellten Beispiele näher beschrieben und erläutert. Die der Beschreibung und den Zeichnungen zu entnehmenden Merkmale können einzeln für sich oder zu mehreren in beliebiger Kombination erfindungsgemäß angewandt werden. Es zeigen:
Figur 1 eine schematische Ansicht einer erfindungsgemäßen Messanordnung mit einer Messvorrichtung,
Figur 2 eine erste perspektivische Ansicht der Messvorrichtung in Figur 1,
Figur 3 eine weitere perspektivische Ansicht der Messvorrichtung gemäß Figur 2,
Figur 4 eine schematische Seitenansicht der Messvorrichtung gemäß Figur 2,
Figur 5 eine schematisch vergrößerte Ansicht eines unteren Teils der Messvorrichtung gemäß Figur 2 mit einer ersten Messeinrichtung,
Figur 6 eine schematisch vergrößerte Ansicht eines oberen Teils der Messvorrichtung gemäß Figur 2 mit einer dritten Messeinrichtung,
Figur 7 eine weitere schematische Seitenansicht des oberen Teils der Messvorrichtung gemäß Figur 6,
Figur 8 eine schematisch vergrößerte Ansicht der Messvorrichtung mit einer zweiten Messeinrichtung,
Figur 9 eine schematische Schnittansicht einer alternativen Ausführungsform eines unteren Teils der Messvorrichtung gemäß Figur 5,
Figur 10 eine perspektivische Ansicht auf ein Blattfederelement,
Figur 11 eine perspektivische Ansicht auf eine alternative Ausführungsform der Blattfederelemente mit einer Haltevorrichtung für die Messvorrichtung gemäß Figur 2,
Figur 12 eine schematische Seitenansicht einer alternativen Ausführungsform der Messvorrichtung gemäß Figur 2,
Figur 13 eine schematische Seitenansicht einer weiteren alternativen Ausführungsform der Messvorrichtung gemäß Figur 2,
Figur 14 eine schematische Ansicht von unten auf ein Druckmembranelement einer Haltevorrichtung für die Messvorrichtung gemäß Figur 13,
Figur 15 eine perspektivische Ansicht einer weiteren alternativen Ausführungsform der Messvorrichtung gemäß Figur 2,
Figur 16 eine weitere perspektivische Ansicht auf die alternative Messvorrichtung gemäß Figur 15, und
Figur 17 eine schematische Schnittansicht der magnetischen Übertragungseinrichtung der alternativen Messvorrichtung gemäß Figur 15.

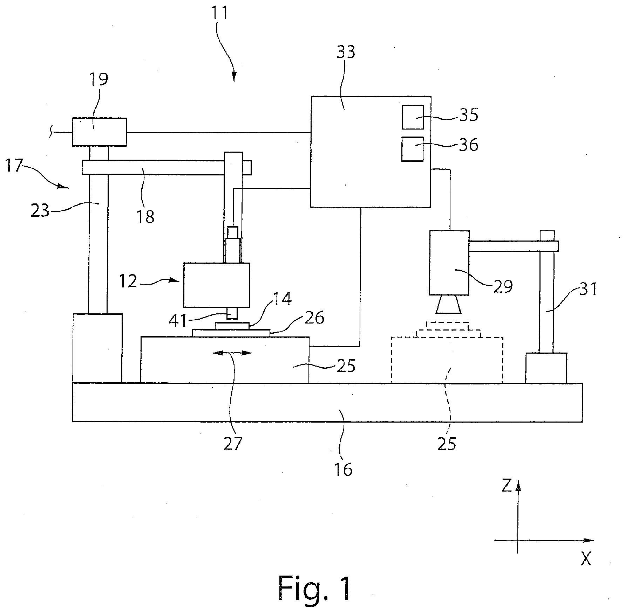

In Figur 1 ist schematisch eine Messanordnung 11 dargestellt. Eine solche Messanordnung 11 kann zum Prüfen von mechanischen und/oder physikalischen Eigenschaften von Oberflächen an Prüfkörpern 14, wie beispielsweise Folien, Schichten und/oder Beschichtungen auf Gegenständen, vorgesehen sein. Beispielsweise kann die Messanordnung 11 als eine Härtemesseinrichtung eingesetzt werden, bei der durch Eindringen mittels eines Eindringkörpers 41 einer Messvorrichtung 12 eine Härtemessung durchgeführt wird. Des Weiteren kann diese Messanordnung 11 mit der Messvorrichtung 12 dafür vorgesehen werden, um eine Kratzfestigkeit einer Folie, einer Schicht oder Beschichtung auf Gegenständen zu ermitteln. Dabei können beispielsweise CVD- oder PVD-Beschichtungen bezüglich deren Kratzfestigkeit getestet werden. Ebenso können weitere Mikrokratzer erfasst oder anderweitige Verformungsinformationen von der Oberfläche erfasst und analysiert werden. Ebenso ermöglicht diese Messanordnung 11 insbesondere mit der Messvorrichtung 12 auch eine Rauheitsmessung einer Oberfläche des Prüfkörpers 14, ohne dass eine Beschädigung der Oberfläche des Prüfkörpers 14 damit einhergeht. In diesem Fall wird der Eindringkörper 41 auf der Oberfläche des Prüfkörpers 14 aufgesetzt und entlang der Oberfläche zum Abtasten der Rauheit der Oberfläche des Prüfkörpers 14 verfahren.

Die Messanordnung 11 umfasst einen gemeinsamen Grundkörper 16. Dieser kann bevorzugt aus Granit ausgebildet sein. Auf dem Grundkörper 16 ist ein Stativ 17 vorgesehen, welches an einem Ausleger 18 die Messvorrichtung 12 aufnimmt. Dieses Stativ 12 umfasst einen Antriebsmotor 19, durch welchen die Messvorrichtung 12 aus einer in Figur 1 dargestellten Ausgangsposition 21 in eine Prüfposition 22 verfahrbar ist, in welcher der Eindringkörper 41 auf einem Prüfkörper 14 aufliegt. Beispielsweise kann der Antriebsmotor 19 den Ausleger 18 für eine auf und ab Bewegung entlang einer Führungssäule 23 des Stativs 12 antreiben.

Auf dem Grundkörper 16 ist des Weiteren ein Messtisch 25 vorgesehen. Dieser Messtisch 25 weist eine Messtischaufnahme 26 auf, welche zumindest in X-Richtung gemäß Pfeil 27 verfahrbar angetrieben ist. Auf die Messtischaufnahme 26 wird der Prüfkörper 14 aufgelegt und daran befestigt.

Die Messanordnung 11 kann des Weiteren eine optische Erfassungseinrichtung 29 umfassen, welche ebenfalls an dem Stativ 17 oder vorteilhafterweise getrennt dazu an einem weiteren Stativ 31 angeordnet sein kann. Diese optische Erfassungseinrichtung 29 kann benachbart zur Messvorrichtung 12 positioniert sein. Der Messtisch 25 oder die Messtischaufnahme 26 ist dabei derart verfahrbar ausgestaltet, dass der Prüfkörper 14 nach dem Einbringen einer Eindringstelle oder eines Kratzers in die Oberfläche des Prüfkörpers 14 zur optischen Erfassungseinrichtung 29 verfahrbar ist, damit die Eindringstelle oder der eingebrachte Kratzer in die Oberfläche des Prüfkörpers 14 optisch erfasst werden kann. Alternativ kann auch eine Verfahrbewegung der Messvorrichtung 12 und der optischen Erfassungseinrichtung 29 relativ zum Messtisch 25 vorgesehen sein.

Die Messanordnung 11 umfasst des Weiteren eine schematisch dargestellte Steuerung 33, welche eine nicht näher dargestellte Datenverarbeitungseinrichtung, eine Anzeigevorrichtung 35 und eine Eingabevorrichtung 36 umfasst. Die Steuerung 33 ist zumindest durch Signalleitungen mit dem Stativ 17, der Messvorrichtung 12 und dem Messtisch 25 verbunden. Bevorzugt ist auch die optische Erfassungseinrichtung 29 und gegebenenfalls das die optische Erfassungseinrichtung 29 aufnehmende Stativ 31 daran angeschlossen.

Die Messanordnung 11 weist des Weiteren zur Ansteuerung der Messvorrichtung 12 zumindest eine Steuerleitung auf, die mit der Steuerung 33 verbunden ist.

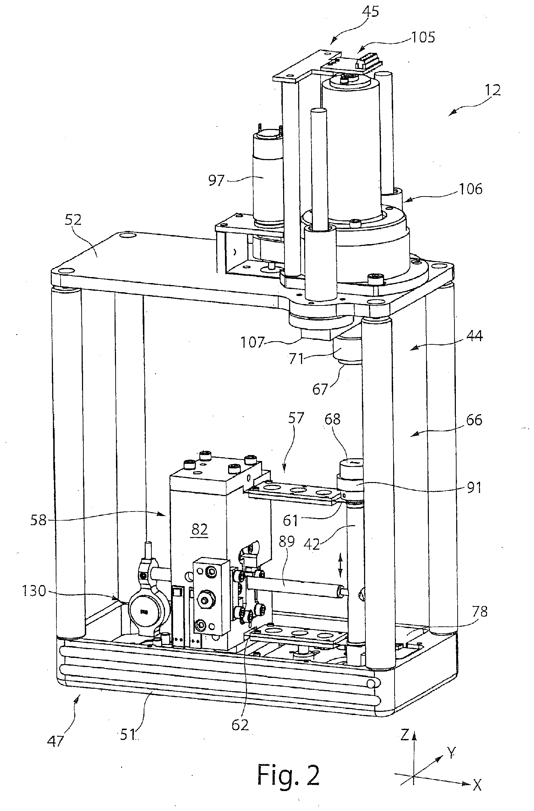

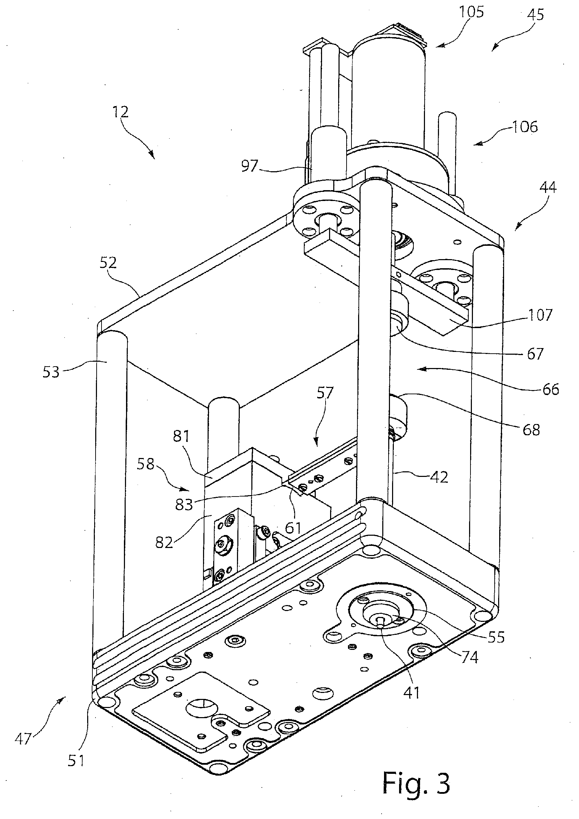

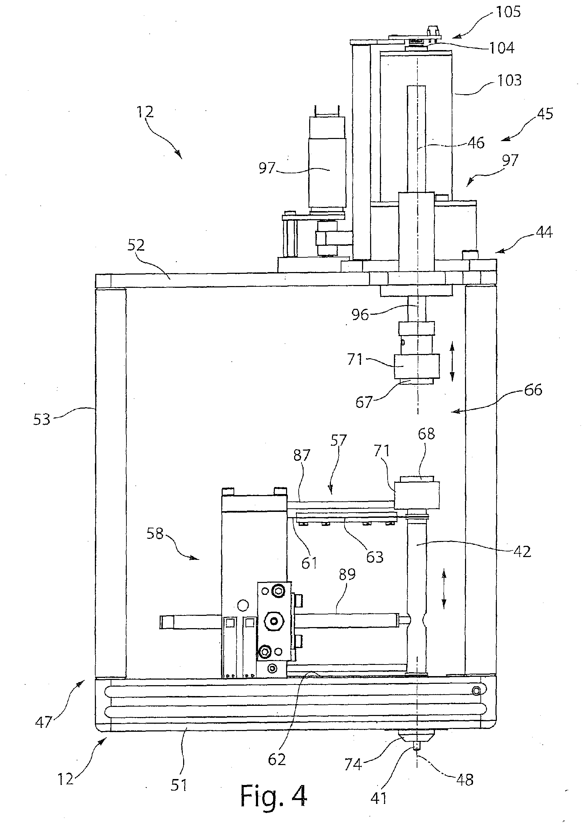

In Figur 2 ist eine erste perspektivische Ansicht der erfindungsgemäßen Messvorrichtung 12 dargestellt. Figur 3 zeigt eine weitere perspektivische Ansicht von unten auf die Messvorrichtung 12 gemäß Figur 2. In Figur 4 ist eine schematische Seitenansicht der Messvorrichtung 12 gemäß Figur 2 dargestellt, auf welche zur Darlegung des Aufbaus der Messvorrichtung 12 ebenfalls Bezug genommen wird.

Die Messvorrichtung 12 umfasst ein Gehäuse 47 mit einer Grundplatte 51. Dieser gegenüberliegend ist ein Deckelelement 52 vorgesehen. Zwischen der Grundplatte 51 und dem Deckelelement 52 sind Abstandselemente 53 vorgesehen. Die das Gehäuse 47 schließenden Seitenwände zwischen der Grundplatte 51 und dem Deckelelement 52 sind zur Wahrung der Übersichtlichkeit nicht dargestellt.

Die Grundplatte 51 weist eine Ausnehmung 55 auf, durch welche sich ein Eindringkörper 41 erstreckt und nach unten austreten kann, wie dies in Figur 3 dargestellt ist. Der Eindringkörper 41 wird von einem Übertragungselement 42 aufgenommen. Dieses ragt in einen Innenraum des Gehäuses 47. Das Übertragungselement 42 ist bevorzugt durch eine Führung 57 innerhalb des Gehäuses 47 aufgenommen. Durch diese Führung 57 ist das Übertragungselement 42 entlang einer Längsachse 43 des Übertragungselementes 42 auf und ab bewegbar. Die Längsachse 43 des Übertragungselementes 42 entspricht einer Längsachse 48 des Eindringkörpers 41.

Die Führung 57, welche das Übertragungselement 42 aufnimmt, ist an einer Haltevorrichtung 58 angeordnet, welche auf der Grundplatte 51 befestigt ist. Die Führung 57 umfasst beispielsweise ein erstes und zweites Blattfederelement 61, 62, welche senkrecht zur Längsachse 43 des Übertragungselements 42 ausgerichtet sind. Die Längsachse 43 des Übertragungselementes 42 liegt vorzugsweise in einer Verfahrachse 46 eines Antriebselementes 96 der Antriebseinrichtung 45 oder ist parallel dazu ausgerichtet. Die Blattfederelemente 61, 62 sind innerhalb des Gehäuses 12 bevorzugt in X-Richtung ausgerichtet, wodurch das Übertragungselement 42 in Z-Richtung ausgerichtet gehalten wird. Durch diese Blattfederelemente 61, 62 wird eine Auf-und-ab-Bewegung beziehungsweise eine Verfahrbewegung entlang der Z-Achse des Gehäuses 47 ermöglicht. Gemäß einer ersten Ausführungsform ist vorgesehen, dass die Blattfederelemente 61, 62 aus einem dünnen flachen Streifen ausgebildet sind, insbesondere einem Federstahl. Zur Versteifung beispielsweise des oberen Blattfederelementes 61 sind an einer Ober- und Unterseite des Blattfederelementes 61, 62 Versteifungselemente 63 befestigt. Diese Versteifungselemente 63 können ebenfalls streifenförmig ausgebildet sein. Vorzugsweise sind diese durch eine Schraub- oder Klipsverbindung fest am Blattfederelement 61 angeordnet. Alternativ kann das obere Blattfederelement 61 auch dickeralso versteifter - ausgebildet sein, so dass die Versteifungselemente 63 entbehrlich sind.

Die Messvorrichtung 12 weist des Weiteren eine Krafterzeugungseinrichtung 44 auf, welche aus einer Antriebseinrichtung 45 besteht, die beispielsweise am Deckelelement 52 befestigt ist.

Des Weiteren umfasst die Krafterzeugungseinrichtung 44 eine magnetische Übertragungseinrichtung 66, welche zumindest einen ersten und zweiten Magnetpol 67, 68 aufweist. Ein erster Magnetpol 67 ist der Antriebseinrichtung 45 zugeordnet. Der zumindest eine zweite Magnetpol 68 ist an einem dem Eindringkörper 41 gegenüberliegenden Ende des Übertragungselementes 42 angeordnet. Der erste und zweite Magnetpol 67, 68 liegen in einer gemeinsamen Längsachse, insbesondere in einer Verfahrachse 46 des Antriebselementes 96, welches bevorzugt in einer Z-Achse des Gehäuses liegt. Der erste und zweite Magnetpol 67, 68 sind derart zueinander ausgerichtet, dass diese mit einem gleichen Pol aufeinander zuweisen. Dadurch ist zwischen den Magnetpolen 67, 68 eine abstoßende Wirkung gegeben. Die abstoßende Wirkung beziehungsweise die Magnetkraft nimmt bei einem sich verringernden Abstand der beiden Magnetpole 67, 68 zueinander zu. Bevorzugt sind die Magnetpole 67, 68 als Permanentmagnet ausgebildet. Durch die magnetische Übertragungseinrichtung 66 ist eine berührungsfreie Kraftübertragung vom Antriebselement 96 der Antriebseinrichtung 45 auf den Eindringkörper 41 ermöglicht. Diese magnetische Übertragungseinrichtung 66 kann auch als magnetische Feder bezeichnet werden. Durch die gegenpolig aufeinander zuweisenden Magnetpole 67, 68 wird eine Verfahrbewegung bei einer Zustellbewegung des Antriebselements 96 auf das Übertragungselement 42 erzeugt. Allerdings ist keine starre Kopplung gegeben, so dass eine Überbelastung in den die Verfahrbewegung des Eindringkörpers 41 erzeugenden Komponenten verhindert ist.

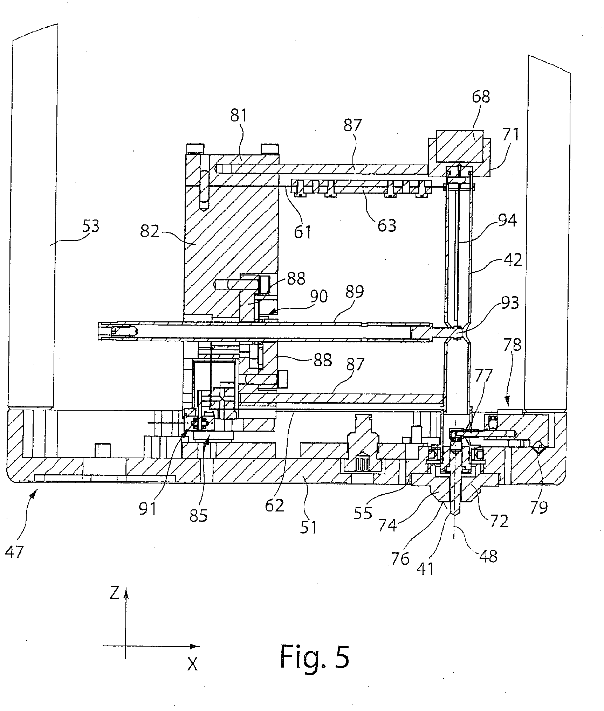

Zur weiteren Veranschaulichung des den Eindringkörper 41 aufnehmenden Übertragungselementes 42 sowie des Aufbaus der Haltevorrichtung 58 und der Ausgestaltung der Führung 47 wird auf Figur 5 Bezug genommen.

Das Übertragungselement 42 ist bevorzugt als ein Rohr ausgebildet. An dessen oberen Ende ist eine Aufnahmeeinrichtung 71 vorgesehen, welche den zweiten Magnetpol 68 aufnimmt. Hierbei kann es sich um ein topfförmiges Element, vorzugsweise aus Kunststoff, handeln. Der Magnetpol 68 kann beispielsweise eingeklebt oder eingepresst sein und ist seitlich in der Aufnahmeeinrichtung 71 geführt. Der Magnetpol 68 ist vorzugsweise zylindrisch ausgebildet. Die Längsachse des Magnetpols 68 ist bevorzugt zur Längsache 43 des Übertragungselementes 42 ausgerichtet. Analoges gilt für den ersten Magnetpol 67. Am gegenüberliegenden Ende des Übertragungselementes 42 ist der Eindringkörper 41 vorgesehen. Dieser ist durch eine Befestigungseinrichtung 72 auswechselbar aufgenommen. Die Befestigungseinrichtung 72 kann bei der Ausgestaltung der Messvorrichtung 12 als Härtemessvorrichtung lediglich durch eine Rast- oder Klipsverbindung vorgesehen sein, so dass eine axiale Sicherung des Eindringkörpers 41 in der Befestigungseinrichtung 72 gegeben ist. Bei der Verwendung der Messvorrichtung 12 für die Ermittlung der Kratzfestigkeit weist die Befestigungsvorrichtung 72 zusätzlich zur axialen Sicherung auch eine radiale Verspannung auf. Dies kann durch eine Gewindeschraube oder dergleichen vorgesehen sein. Insbesondere kann die Befestigungseinrichtung 72 als Spannzangensystem ausgebildet sein.

Das untere Ende des Übertragungselements 42 taucht in die Ausnehmung 55 der Grundplatte 51 berührungsfrei ein. In dieser Ausnehmung 55 ist ein Aufsetzring 74 positioniert, durch welchen der Eindringkörper 41 frei und ohne Reibung hindurchgeführt ist, so dass dessen Spitze frei nach unten austreten kann. Die Spitze des Eindringkörpers 41 wird in Abhängigkeit der durchzuführenden Messung ausgewählt. Diese kann kegelstumpfförmig oder pyramidenförmig sein. Im Falle der Durchführung einer Messung der Kratzfestigkeit ist der Eindringkörper 41 in der Befestigungseinrichtung 72 spezifisch ausgerichtet.

Am inneren Ende des Eindringkörpers 41 ist ein Messfühler 77 einer ersten Messeinrichtung 78 vorgesehen. Dieser ragt durch eine Öffnung im Übertragungselement 42 in das Übertragungselement 42 hinein. Diese erste Messeinrichtung 78 ist vorzugsweise als Abstandssensor ausgebildet und an der Grundplatte 51 befestigt. Die Einstellung eines Abstands des Messfühlers 77 zum inneren Ende des Eindringkörpers 51 ist durch eine Verstellanordnung 79 möglich. Durch diese erste Messeinrichtung 78 wird ein Abstand des Eindringkörpers 41 zum Messfühler 47 ausgehend von einer Ausgangsposition zu einer Eindringposition erfasst und an die Steuerung 33 weitergegeben.

Durch die Führung 57 beziehungsweise die zwei parallel zueinander beabstandeten Blattfederelemente 61, 62 kann eine geführte Auf-und-ab-Bewegung des Übertragungselementes 42 und somit des Eindringkörpers 41 entlang der Z-Achse beziehungsweise der Verfahrachse 46 erzielt werden. Das obere Blattfederelement 61 ist an der Haltevorrichtung 58 geklemmt gehalten. Beispielsweise ist eine Befestigungsplatte 81 durch eine lösbare Verbindung, insbesondere Schraubverbindung, an einem Montageblock 82 befestigt. Zur definierten Ausrichtung des Blattfederelements 61 im Montageblock 82 kann eine Vertiefung 83 vorgesehen sein, durch welche das Blattfederelement 61 entlang einer X-Achse des Gehäuses 47 ausgerichtet ist.

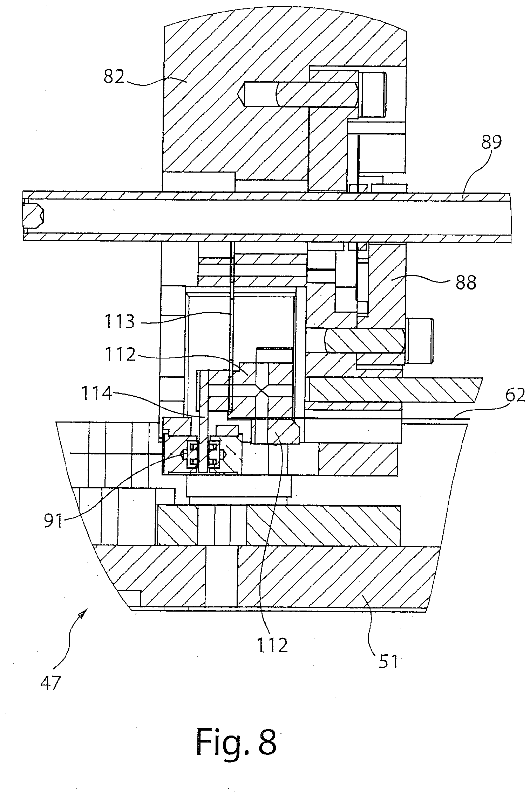

Das untere Blattfederelement 62 ist mittels einer Spanneinrichtung 85 im Montageblock 82 gelagert. Diese Spanneinrichtung 85 wird nachfolgend in Figur 8 näher beschrieben.

In Figur 5 ist des Weiteren eine Transportsicherung 87 durch zwei an der Haltevorrichtung 58 angeordnete Stäbe dargestellt. Der obere Stab ist mit sehr geringem Abstand zur Aufnahmeeinrichtung 71 angeordnet. Der untere Stab 87 endet mit geringem Abstand vor dem Übertragungselement 42. Somit werden beim Transport bereits geringe Auslenkungen blockiert.

Des Weiteren sind im Montageblock 82 zwei gegenläufig ausgerichtete U-förmige Platten 88 vorgesehen, welche ein Ausgleichselement 89 in einer quasi horizontalen Ausrichtung beziehungsweise Ausrichtung in X-Richtung beim Transport sichern können.

Dieses Ausgleichselement 89 kann ergänzend vorgesehen sein. Bei der Ausgestaltung der Messvorrichtung 12 als reines Härtemessgerät ist dieses Ausgleichselement 89 nicht erforderlich. Zur Ermittlung der Kratzfestigkeit kann dadurch eine weitere Aussteifung geschaffen werden, die einer Auslenkbewegung des Eindringkörpers 41 entgegenwirkt. Das Ausgleichselement 89 ist im Montageblock 82 drehbar gelagert. Vorzugsweise ist eine Spannbandlagerung 90 vorgesehen, durch welche eine schwenkbare Anordnung des Ausgleichselements 89 um die Y-Achse im Gehäuse 47 ermöglicht ist. Ein zum Übertragungselement 42 weisendes Ende 93 ragt vorzugsweise durch eine Öffnung in das Übertragungselement 42 hinein. An diesem Ende 93 greift ein weiteres Blattfederelement 94 an, dessen gegenüberliegendes Ende am oberen Ende des Übertragungselementes 42 fixiert ist. Dieses Blattfederelement 94 kann wiederum versteift ausgebildet sein. Bevorzugt ist das Ausgleichselement 89 rohrförmig ausgebildet.

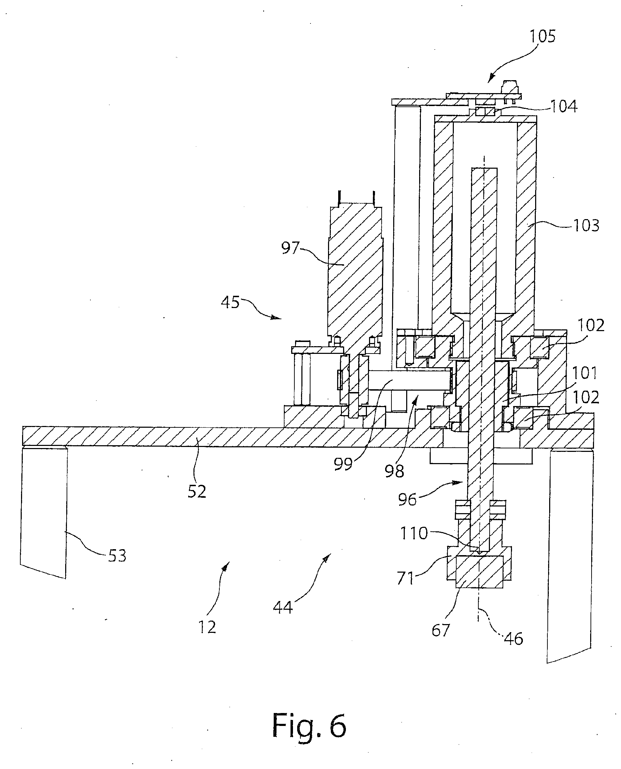

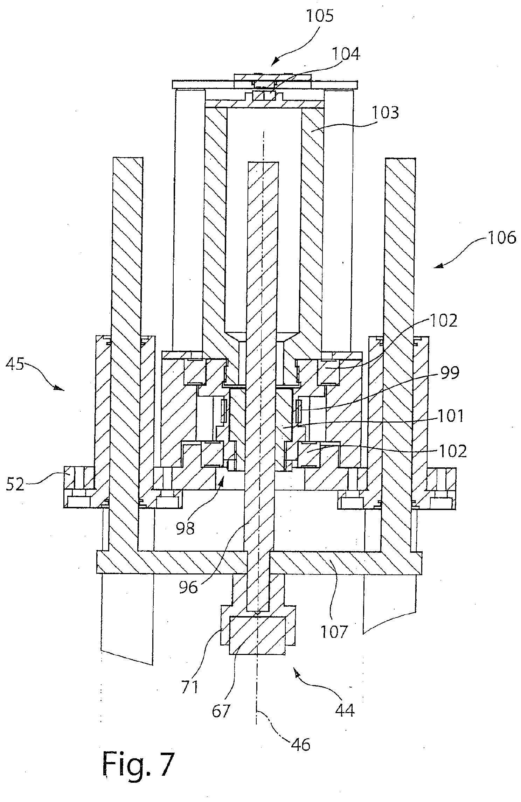

Die Antriebseinrichtung 45 ist schematisch vergrößert in einer ersten Seitenansicht in Figur 6 und in einer zweiten Seitenansicht um 90° gedreht zur ersten Seitenansicht in Figur 7 dargestellt. Die Antriebseinrichtung 45 weist ein Antriebselement 96 auf, welches insbesondere als Antriebsspindel ausgebildet ist. An einem unteren freien Ende des Antriebselementes 95 ist eine Aufnahmeeinrichtung 71 zur Aufnahme des ersten Magnetpols 67 vorgesehen. Die Aufnahmeeinrichtung 71 für den ersten und zweiten Magnetpol 67, 68 sind vorzugsweise identisch. Die Anordnungen des ersten und zweiten Magnetpols 67, 68 können auch vertauscht sein.

In der Aufnahmeeinrichtung 71, welche einerseits am Übertragungselement 42 und andererseits am Antriebselement 96 vorgesehen ist, kann jeweils ein Magnetpol 67, 68 vorgesehen sein. Des Weiteren kann die Aufnahmeeinrichtung 71 derart gestaltet sein, dass mehrere einzelne Magnetpole darin anordenbar sind. Auch können die Magnetpole anstelle einer Klebeverbindung durch eine Rast- oder Klipsverbindung, beispielsweise durch ein zusätzliches Verschlusselement, welches an der Aufnahmeeinrichtung 71 angreift, gehalten werden.

Die Magnetpole 67, 68 sind vorteilhafterweise zylinderförmig ausgebildet. Weitere Geometrien sind ebenso möglich. Darüber hinaus können die Magnetpole 67, 68 auch als Ringe mit einer inneren Durchgangsbohrung ausgebildet sein.

Zur Ansteuerung einer Verfahrbewegung des Antriebselementes 96 entlang der Verfahrachse beziehungsweise der Z-Achse des Gehäuses 47 ist ein Antriebsmotor 97 vorgesehen. Vorteilhafterweise ein Elektromotor, insbesondere Servomotor, vorgesehen. Dieser Antriebsmotor 97 treibt einen Drehantrieb 98 an, welcher den Antriebsmotor 97 mit dem Antriebselement 96 verbindet. Der Drehantrieb 98 umfasst beispielsweise einen Zahnriemen 99, der an einem Ritzel an einer Antriebswelle des Antriebsmotors 97 und gegenüberliegend an einer drehbar gelagerten Spindelmutter 101 antreibt. Die Spindelmutter 101 ist drehbar mittels eines Lagers 102 aufgenommen. An der Spindelmutter 101 ist eine Hülse 103 drehfest vorgesehen, welche an einem oberen Ende eine Komponente 104 einer dritten Messeinrichtung 105 aufnimmt. Die dritte Messeinrichtung 105 ist feststehend mit dem Gehäuse 47 verbunden. Bevorzugt ist die dritte Messeinrichtung 105 als ein Drehgeber oder Inkrementalgeber ausgebildet, durch welchen die durchgeführte Umdrehung der Spindelmutter 101 ermittelt wird.

Zur drehgesicherten Auf-und-ab-Bewegung des Antriebselementes 96 ist eine Säulenführung 106 vorgesehen. Diese Säulenführung ist am Deckelelement 52 befestigt und umfasst eine U-förmige Führungssäule 107.

Bevorzugt ist zwischen dem Magnetpol 67 und der Aufnahmeeinrichtung 71 oder zwischen der Aufnahmeeinrichtung 71 und dem Antriebselement 96 eine vierte Messeinrichtung 110 vorgesehen, welche vorzugsweise als Kraftsensor ausgebildet ist. Diese zweite Messeinrichtung 110 erfasst die zwischen den beiden Magnetpolen 67, 68 wirkende Kraft. Dadurch kann eine weitere überwachende Messgröße zur Ermittlung von Messergebnissen bereitgestellt werden. Insbesondere kann eine Überwachung und gegebenenfalls eine Korrektur der Eindringkraft ermittelt werden. Durch eine Zustellbewegung des Antriebselements 96, dessen Verfahrweg durch die dritte Messeinrichtung 105 erfasst wird, kann aufgrund der bekannten Magnetkraft der beiden Magnetpole 67, 68 die auf den Eindringkörper 41 übertragende Kraft errechnet werden. Ein Abgleich bezüglich der tatsächlich wirkenden Kraft ist durch die vierte Messvorrichtung 110 möglich.

In Figur 8 ist schematisch vergrößert die Spanneinrichtung 85 dargestellt. Das untere Blattfederelement 62 ist an dem Übertragungselement 42 gegenüberliegenden Ende durch zwei Spannelemente 112 geklemmt gehalten. Diese beiden Spannelemente 112 sind wiederum durch ein weiteres Blattfederelement 113 an dem Montageblock 82 gehalten, wobei das Blattfederelement 113 in Z-Richtung ausgerichtet ist. Dadurch ist eine Auslenkbewegung des unteren Blattfederelementes 62 in und entgegen der X-Richtung möglich. An dem einen Spannelement 112 ist des Weiteren eine Fahne 114 vorgesehen, welche in eine zweite Messvorrichtung 91 eintaucht. Diese Messvorrichtung 91 ist wiederum als Abstandssensor ausgebildet. Durch eine Verlagerung der Fahne 114 kann durch die zweite Messvorrichtung 91 eine auf das Blattfederelement 62 wirkende Kraft oder Verfahrbewegung erfasst werden. Diese Kraft- oder Verfahrbewegung wird über den Eindringkörper 41 und das Übertragungselement 42 auf das Blattfederelement 62 übertragen. Insbesondere bei der Durchführung einer Messung der Kratzfestigkeit kann durch diese zweite Messeinrichtung 91 ein weiterer Parameter bezüglich der Auslenkung des Eindringkörpers 41 erfasst werden.

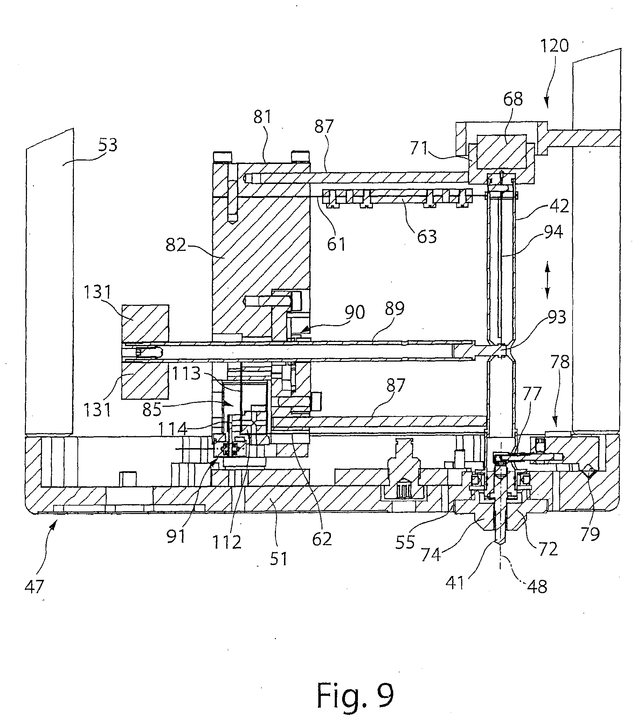

In Figur 9 ist eine schematische Schnittansicht eines unteren Teils der Messvorrichtung 12 gemäß einer alternativen Ausführungsform zu Figur 5 dargestellt. Diese Ausführungsform gemäß Figur 9 weicht dahingehend von der Ausführungsform in Figur 5 ab, dass beispielsweise dem zweiten Magnetpol 68 eine Schwingungsdämpfungseinrichtung 120 zugeordnet ist. Anstelle dieser dem Magnetpol 68 zugeordnete Schwingungsdämpfungseinrichtung 120 kann eine Schwingungsdämpfungseinrichtung 130 auch an einem dem Übertragungselement 42 gegenüberliegenden Ende des Ausgleichselementes 89 vorgesehen sein. Eine Kombination beider ist ebenfalls möglich. Die Schwingungsdämpfungseinrichtungen 120, 130 haben zur Aufgabe, dass unmittelbar nach einem Aufsetzen des Eindringkörpers 41 auf einer Oberfläche des Prüfkörpers 14 einer oder mehreren Abhebebewegungen des Eindringkörpers 41 entgegengewirkt wird. Bevorzugt werden sogenannte Wirbelstrombremsen eingesetzt.

Die Schwingungsdämpfungseinrichtung 120 ist beispielsweise durch eine Umhausung 121 gebildet, welche vorzugsweise als Rohrabschnitt ausgebildet ist. Diese umgibt den Magnetpol 68. Bevorzugt ist der Magnetpol 68 zumindest teilweise innerhalb der Umhausung 121 in einer Ausgangsposition positioniert. Sobald eine Abhebebewegung in Richtung der Z-Achse beziehungsweise der Längsachse 43 erfolgt, taucht der Magnetpol 68 in die Umhausung 121 ein, wodurch eine entgegenwirkende Magnetkraft verstärkt ist. Die Umhausung 121 ist bevorzugt aus ferromagnetischem Material, insbesondere Kupfer, ausgebildet. Die Umhausung 121 ist bevorzugt in der Höhe zum Magnetpol 68 einstellbar. Bevorzugt kann die Umhausung 121 in der Höhe entlang des Abstandselements 53 verfahren werden.

Die Schwingungsdämpfungseinrichtung 130 ist lediglich teilweise dargestellt. Eine aus ferromagnetischem Material ausgebildete Fahne 131 ist am Ausgleichselement 89 vorgesehen. Diese Fahne 131 ist zwischen zwei zueinander beabstandeten Permanentmagneten positioniert, um wiederum als magnetische Wirbelstrombremse zu wirken.

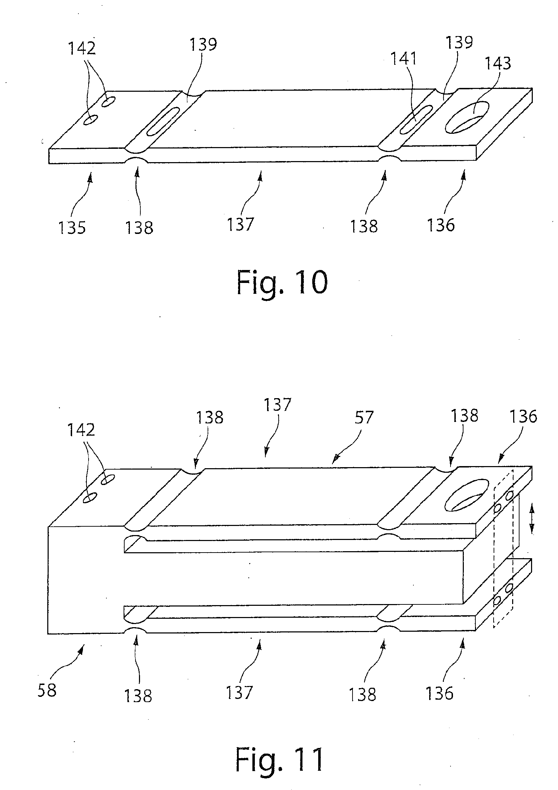

In Figur 10 ist schematisch vergrößert eine alternative Ausführungsform eines Blattfederelements 61, 62 dargestellt. Dieses Blattfederelement 61, 62 erfordert kein Versteifungselement 63. Vielmehr ist der konstruktive Aufbau so gewählt, dass dieses entbehrlich ist. Das Blattfederelement 61 weist einen Klemmbereich 135 sowie einen Anschlussbereich 136 und einen dazwischenliegenden Distanzabschnitt 137 auf. Zwischen dem Klemmbereich 135 und dem Distanzabschnitt 137 sowie zwischen dem Distanzabschnitt 137 und dem Anschlussbereich 136 ist ein Festkörpergelenk 138 ausgebildet. Dieses Festkörpergelenk 138 ist in der Dicke gegenüber dem Klemmbereich 135, Anschlussbereich 136 und dem Distanzelement 137 reduziert. Dadurch wird ein Scharnier gebildet. Zur Einstellung der Steifigkeit des Festkörpergelenkes 138 dient einerseits die Reduzierung in der Dicke als auch die Ausgestaltung der Radien 139. Ergänzend können ein oder mehrere Langlochausnehmungen 141 vorgesehen sein, um weichere Festkörpergelenke 138 auszubilden. Der Distanzabschnitt 137 kann anstelle einer vollflächigen Ausgestaltung auch als ein Rahmen oder eine Tragstruktur ausgebildet sein. Zur Ausrichtung des Blattfederelementes 61 an der Haltevorrichtung 58 sind beispielsweise Bohrungen 142 vorgesehen, um den Klemmbereich 135 mit dem Montageblock 82 zu verstiften. Gegenüberliegend im Anschlussbereich 136 ist eine Aufnahmebohrung 143 vorgesehen, in welcher das Übertragungselement 42 positionierbar ist. Vorzugsweise sind der Anschlussbereich 136 und das Übertragungselement 42 durch eine Klebeverbindung miteinander verbunden.

In Figur 11 ist eine alternative Ausgestaltung der Führung 57 und der Haltevorrichtung 58 ausgebildet. Bei dieser Ausführungsform ist vorgesehen, dass die Führung 57 und Haltevorrichtung 58 einteilig sind. Durch eine Bearbeitung der Führung 57 aus einem Werkstückkörper, beispielsweise durch Erodieren oder Feinstbearbeitung, ist ermöglicht, dass der Klemmbereich 135 einteilig mit der Haltevorrichtung 58, insbesondere dem Montageblock 82, verbunden sind und dennoch die Blattfederelemente 61, 62 mit den Festkörpergelenken 138, dem Distanzabschnitt 137 und dem Anschlussbereich 136 ausgebildet sind. Zwischen den Blattfederelementen 61, 62 erstreckt sich ein Stützkörper 146. Dieser begrenzt eine Auslenkbewegung des Übertragungselementes 42 entlang der Längsachse 43, 48 beziehungsweise in Z-Richtung. Die beiden stirnseitigen Enden der Anschlussbereiche 136 sind fest mit dem Übertragungselement 42 verbunden, so dass sowohl eine Bewegung nach oben als auch nach unten - also in und entgegen der Z-Richtung - begrenzt ist.

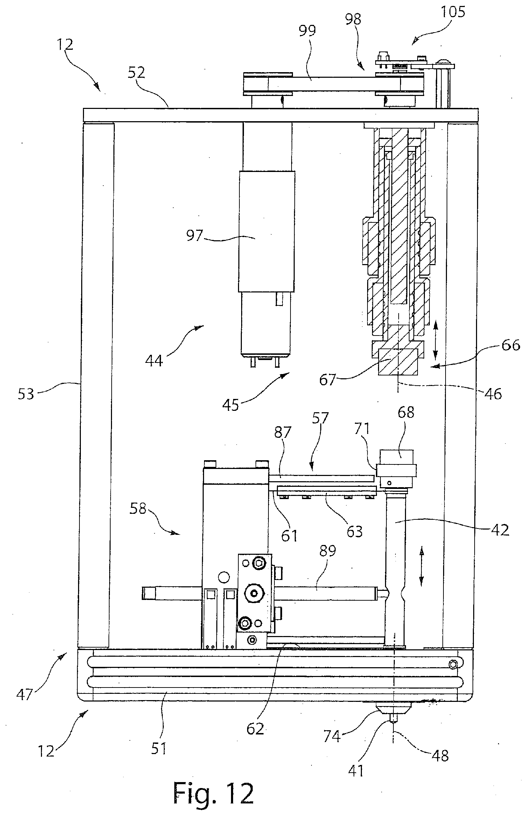

In Figur 12 ist eine schematische Seitenansicht einer alternativen Ausführungsform der Messvorrichtung 12 zu der vorbeschriebenen Messeinrichtung dargestellt. Diese Messvorrichtung 12 ist im Aufbau und der Ausgestaltung bezüglich der Antriebseinrichtung 45 abweichend zu dem insbesondere in den Figuren 6 und 7 beschriebenen Aufbau ausgebildet. Der Antriebsmotor 97 der Antriebsvorrichtung 45 ist nicht außerhalb, sondern innerhalb eines Grundkörpers 16 der Messeinrichtung 12 vorgesehen. Der Antriebsmotor 97 ist also innenliegend angeordnet, das heißt an einer Unterseite des Deckelelementes 52 positioniert. Ebenfalls ist an einer Unterseite des Deckelelementes 52 das Antriebselement 96 befestigt und innenliegend. Das Antriebselement 96 ist in dieser Ausführungsform als eine sogenannte Teleskopspindel ausgebildet. Diese Teleskopspindel weist eine zentrale Antriebsspindel auf, welche mittels des Drehantriebs 98 über einen Zahnriemen 99 durch den Antriebsmotor 97 angetrieben ist. Dabei wird eine teleskopartige Ausfahrbewegung der Teleskopspindel erzielt, so dass die an dessen unteren freien Ende angeordnete Aufnahmeeinrichtung 71 mit dem ersten Magnetpol 67 auf den gegenüberliegenden Magnetpol 68 zubewegt wird.

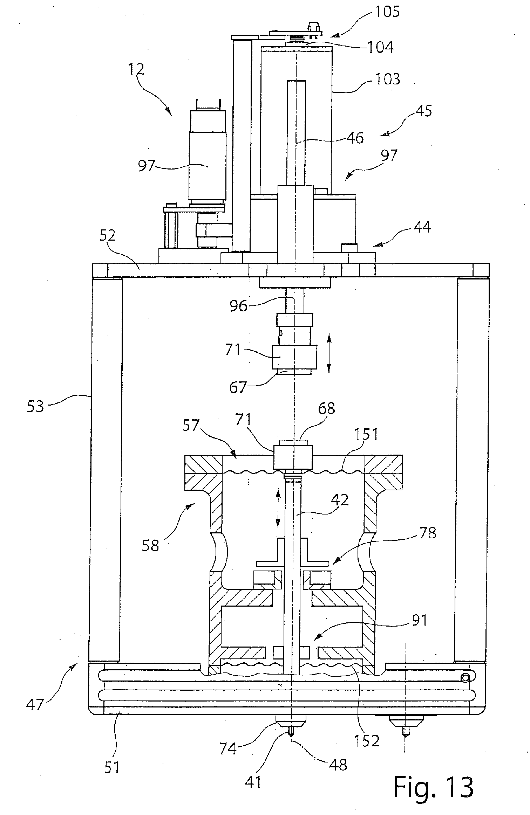

Diese Ausführungsform der Messeinrichtung 12 weist den Vorteil auf, dass die Bauhöhe verringert ist. Der außerhalb des Gehäuses liegende Drehantrieb 98 kann mittels einer nicht näher dargestellten Abdeckung geschützt sein. In Figur 13 ist eine alternative Ausführungsform der Messvorrichtung 12 zu Figur 2 dargestellt. Diese Messeinrichtung 12 weist eine Führung 57 für das Übertragungselement 42 auf, die von den vorbeschriebenen Ausführungsbeispielen abweicht. Bei dieser Ausführungsform ist die Haltevorrichtung 58 vorzugsweise zylindrisch ausgebildet und nimmt am oberen und unteren Ende jeweils ein Druckmembranelement 151, 152 auf. Durch die parallel zueinander beabstandete Anordnung der Druckmembranelemente 151, 152 wird bei der Einwirkung der Magnetkraft über die magnetische Übertragungseinrichtung 66 beziehungsweise den ersten Magnetpol 67 auf den zweiten Magnetpol 68, der über die Aufnahmeeinrichtung 71 an dem Übertragungselement 42 angeordnet ist, eine Verfahrbewegung entlang der Verfahrachse 48 bewirkt. Durch eine solche axiale Verfahrbewegung entlang der Verfahrachse 48 wird der Eindringkörper 41 koaxial gegenüber dem Aufsetzring 74 nach unten beziehungsweise nach außen bewegt. Dadurch kann eine Eindringbewegung des Eindringkörpers 41 in die Messoberfläche des Messgegenstandes erfolgen. Das Druckmembranelement 151, 152 kann in einem Längsschnitt gesehen wellenförmig ausgebildet sein. In der Draufsicht gesehen bedeutet dies, dass konzentrische Kreise vorgesehen sind. Durch die Anzahl und der Höhe der Wellen kann der Freiheitsgrad der Auslenkbewegung beziehungsweise die Auslenkkraft entlang der Verfahrachse 48 bestimmbar sein. Die Druckmembranelemente 151, 152 sind bevorzugt aus nicht-magnetischem Material ausgebildet. Diese bestehen aus einem dünnen scheibenförmigen Federmaterial.

An dem Übertragungselement 42 ist die erste Messeinrichtung 78 vorgesehen, wobei ein Sensorelement der Messeinrichtung 78 fest am Übertragungselement 42und das komplementäre Sensorelement der Messeinrichtung 78 fest an der Haltevorrichtung 58 angeordnet ist. Durch eine Verfahrbewegung entlang der Verfahrachse 48 verändert sich der Abstand zwischen den beiden Sensorelementen, wodurch der Verfahrweg exakt ermittelt werden kann. Diese erste Messeinrichtung 78 arbeitet analog zu der vorstehend beschriebenen ersten Messvorrichtung 78.

An dem Übertragungselement 42 ist bevorzugt eine zweite Messeinrichtung 91 angeordnet. Die zweite Messeinrichtung 91 weist ebenfalls ein Sensorelement unmittelbar am Übertragungselement 42 und benachbart dazu ein an der Haltevorrichtung 58 angeordnetes zweites Sensorelement auf. Durch diese zweite Messeinrichtung 91 kann ein Abweichen in der Auslenkung des Eindringkörpers 41 während einer Verfahrbewegung in X-Richtung oder entgegen der X-Richtung erfasst werden. Die Ausgestaltung der zweiten Messeinrichtung 91 entspricht der in Figur 9 beschriebenen Messeinrichtung 91.

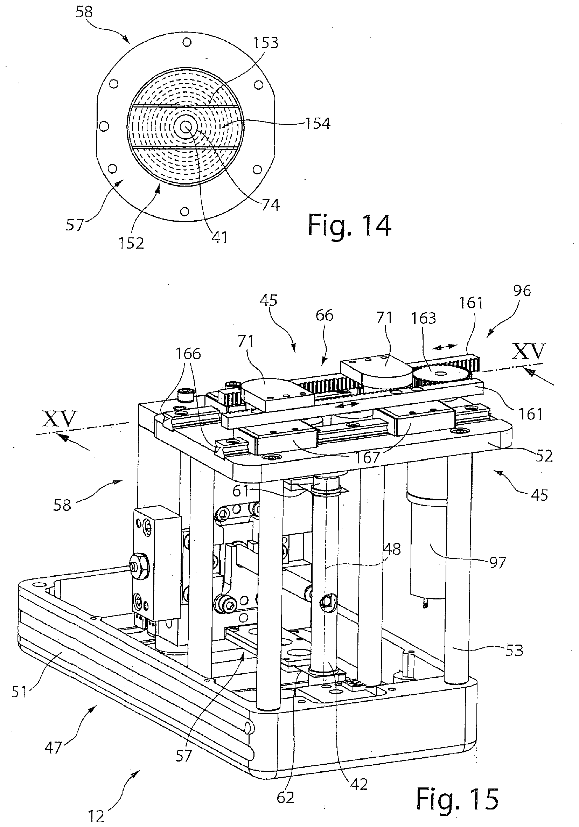

In Figur 14 ist eine schematische Ansicht von unten auf das untere oder das zweite Druckmembranelement 152 dargestellt, welches nahe zum Eindringkörper 41 ausgerichtet ist. Die Haltevorrichtung 58 nimmt das Druckmembranelement 152 vorzugsweise geklemmt auf. Strichliniert sind die konzentrischen Wellen des Druckmembranelementes 152 dargestellt, die auch beim Druckmembranelement 151 vorgesehen sind. Des Weiteren weist dieses untere Druckmembranelement 152 abweichend zum oberen Druckmembranelement 151 zwei Längsschlitze 153 auf, die parallel zueinander beabstandet sind. Die Längsschlitze 152 sind in X-Richtung ausgerichtet beziehungsweise verlaufen parallel zur X-Achse. Dadurch ist das Druckmembranelement 152 entlang der Y-Achse weich beziehungsweise nachgiebig und in der X-Achse steif ausgebildet. Dadurch kann bei der Messung der Kratzfestigkeit eine Auslenkung des Eindringkörpers 41 in X-Richtung erfasst werden.

Bei der Ausführungsform gemäß Figur 13 kann am Übertragungsstift 42 in Analogie zur zweiten Messeinrichtung 91 eine weitere Messeinrichtung vorgesehen sein, die um 90° versetzt angeordnet ist. Dadurch kann während der Messung einer Kratzfestigkeit eine Auslenkbewegung des Eindringkörpers 41 in Y-Richtung erfasst werden.

Im Übrigen wird auf die vorbeschriebenen Ausführungsformen und Alternativen Bezug genommen.

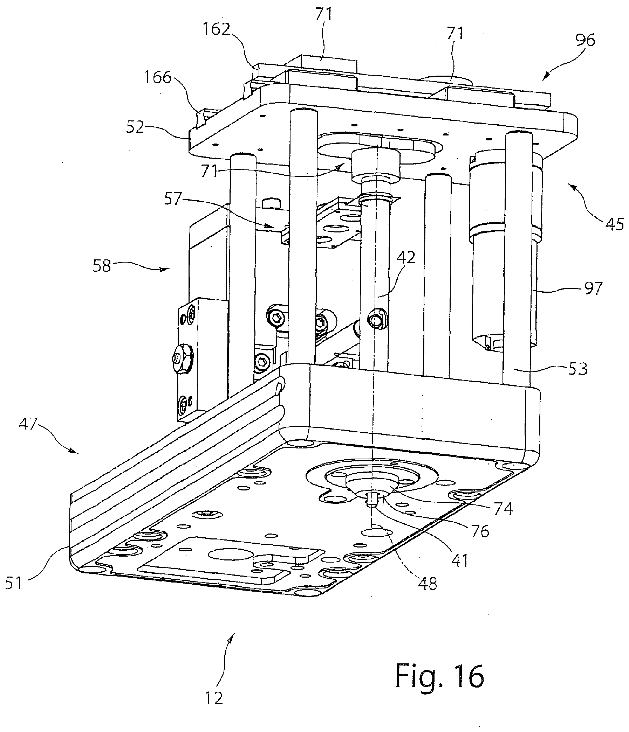

In Figur 15 ist eine erste perspektivische Ansicht einer weiteren alternativen Ausführungsform der Messvorrichtung 12 zu der Ausführungsform in Figur 2 dargestellt. Die Figur 16 zeigt eine weitere perspektivische Ansicht der alternativen Ausführungsform der Messvorrichtung 12 gemäß Figur 15.

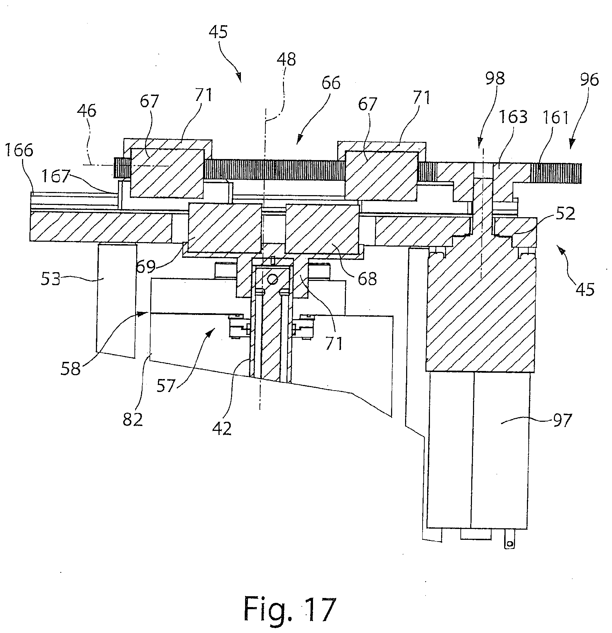

Diese Messvorrichtung 12 weicht gegenüber der ersten Ausführungsform gemäß Figur 2 dahingehend ab, dass die Antriebseinrichtung 45 der Krafterzeugungseinrichtung 44 abweichend ausgebildet ist. In Figur 17 ist eine schematische Schnittansicht dieser alternativen Ausführungsform der Antriebseinrichtung 45 zum besseren Verständnis dargestellt.

Die Antriebseinrichtung 45 dieser alternativen Ausführungsform ist an dem Gehäuse 47, insbesondere dem Deckelelement 52, angeordnet. Der Antriebsmotor 97 treibt ein Antriebselement 96 an, welches durch zwei Zahnstangen 161, 162 gebildet ist, die parallel zueinander ausgerichtet sind. Dieses Paar Zahnstangen 161, 162 wird durch ein Antriebsrad 163 angetrieben, welches wiederum mit dem Antriebsmotor 97 drehverbunden ist. Bevorzugt ist dieses Antriebsrad 163 unmittelbar an der Antriebswelle des Antriebsmotors 97 vorgesehen. Alternativ kann dazwischen auch ein Getriebe für eine Unter- oder Übersetzung vorgesehen sein. Dieses Antriebsrad 163 treibt gleichzeitig beide Zahnstangen 161, 162 an. Dadurch wird eine gegenläufige Verfahrbewegung der Zahnstangen 161, 162 eingeleitet.

Die Drehbewegung der Antriebsachse des Antriebsmotors 97 oder des Antriebsrades 163 kann in einfacher Wiese erfasst oder decodiert werden, so dass daraufhin aufgrund der feststehenden geometrischen Verhältnisse vom Antriebsrad 163 und Zahnstange 161, 162 eine exakte Erfassung und Ansteuerung des Verfahrweges der Permanentmagnete des ersten Magnetpols 67 ermöglicht ist.

Die Verfahrbewegung der Zahnstangen 161, 162 erfolgt entlang einer Verfahrachse 46, welche senkrecht zur Verfahrachse 48 des Übertragungselementes 42 beziehungsweise des Eindringkörpers 41 ausgerichtet ist. Im Ausführungsbeispiel ist die Verfahrachse 48 somit in Z-Richtung - also in der Vertikalen - ausgerichtet und die Verfahrachse 46 in der XY-Ebene beziehungsweise in der Horizontalen.

Das Antriebselement 96 ist durch eine Führung 165 verfahrbar aufgenommen. Vorzugsweise besteht die Führung 165 aus zwei parallel zueinander ausgerichteten Führungsschienen 166, welche einen oder mehrere verfahrbare Schlitten 167 führt. An dem oder den Schlitten 167 ist jeweils eine Zahnstange 161, 162 angeordnet.

Bei dieser alternativen Ausführungsform ist vorgesehen, dass der erste Magnetpol 67 aus zwei separaten Permanentmagneten gebildet ist. Alternativ können auch mehrere separate Permanentmagnete vorgesehen sein. Der zweite Magnetpol 68 ist in der Anzahl der separaten Permanentmagnete an die Anzahl des ersten Magnetpols 67 angepasst. Die Aufnahmeeinrichtung 71 am Übertragungselement 42 weist zwei separate Vertiefungen auf, welche in gleichem Abstand zur Verfahrachse 48 die Permanentmagnete zur Bildung des zweiten Magnetpols 68 aufnehmen.

Die Aufnahmevorrichtung 71 zur Aufnahme des ersten Magnetpols 67 ist durch zwei getrennt zueinander angeordnete Aufnahmeelemente 71 gebildet. Jedes der Aufnahmeelemente 71 für den Permanentmagnet ist an einer Zahnstange 161, 162 angeordnet, wobei diese jeweils derart ausgerichtet sind, dass zwischen den beiden parallel verlaufenden Zahnstangen 161, 162 die Aufnahmeeinrichtung 71 positioniert ist.

In einer Ausgangsposition der Messvorrichtung 12 sind die beiden Permanentmagnete des ersten Magnetpols 67 derart zueinander beabstandet, dass diese keine oder nahezu keine Magnetkraft auf die gegenüberliegend angeordneten Permanentmagnete des zweiten Magnetpols 68 ausüben. Zur Ansteuerung einer Eindringbewegung des Eindringkörpers 41 wird mittels des Antriebsmotors 97 eine Drehbewegung in das Antriebsrad 73 eingeleitet, durch welches die beiden Zahnstangen 161, 162 synchron angetrieben und gegenläufig zueinander verfahren werden. Die beiden Permanentmagnete des ersten Magnetpols 67 werden gleichzeitig aufeinander zubewegt. Bei einer Position der Permanentmagnete des ersten Magnetpols 67 zu denen des zweiten Magnetpols 68, wie dies in Figur 17 dargestellt ist, wird aufgrund des geringen Überdeckungsgrades nur eine geringe Magnetkraft übertragen. Die maximale Kraftübertragung ist dann gegeben, wenn die Permanentmagnete des ersten Magnetpols 67 derart aufeinander zubewegt werden, bis diese deckungsgleich zu den Permanentmagneten des zweiten Magnetpols 68 positioniert sind. Der Grad der Überdeckung wird durch eine Steuerung der Messvorrichtung 12 angesteuert, insbesondere in Abhängigkeit der Eindringbewegung oder des Verfahrweges des Eindringkörpers 41.

Alternativ zur vorstehenden Verfahrbewegung der Permanentmagnete des ersten Magnetpols 67 zur Ansteuerung der Eindringbewegung des Eindringkörpers 41 kann auch vorgesehen sein, dass die Permanentmagnete des ersten Magnetpols 67 in einer Ausgangsposition benachbart aneinander liegen und die Permanentmagnete des zweiten Magnetpols 68 in einem großen Abstand außerhalb der beiden unmittelbar benachbart zueinander angeordneten Permanentmagnete des ersten Magnetpols 67 liegen. In diesem Fall wird eine Verfahrbewegung der Permanentmagnete des ersten Magnetpols 67 voneinander wegführend angesteuert.

In den in den Figuren 15 bis 17 dargestellten Ausführungsformen ist die Verfahrachse 46 beispielsweise in Y-Richtung gemäß dem in Figur 2 dargestellten Koordinatensystem ausgerichtet. Alternativ kann diese Verfahrachse 46 auch in einer weiteren Richtung in der XY-Ebene ausgerichtet sein, insbesondere in der X-Achse.

Gemäß einer weiteren nicht näher dargestellten Ausführungsform der Messvorrichtung 12 gemäß Figur 15 kann auch vorgesehen sein, dass der erste und zweite Magnetpol 67, 68 nur aus einem Permanentmagneten bestehen. Somit genügt die Ansteuerung von nur einem Antriebselement, an welchem der erste Magnetpol 67 befestigt ist, um diesen entlang der Verfahrachse 46 senkrecht zur Verfahrachse 48 des Übertragungselements zu verfahren.

Des Weiteren kann alternativ vorgesehen sein, dass beispielsweise mehr als zwei Permanentmagnete je Magnetpol 67, 68 vorgesehen sind. Diese können dann auf einem Kreisumfang angeordnet werden. Durch eine Schwenkbewegung können somit mehrere Permanentmagnete des ersten Magnetpols 67 gleichzeitig in teilweise oder vollständige Überdeckung zu den jeweiligen Permanentmagneten des zweiten Magnetpols 68 gebracht werden.

Im Übrigen gelten die vorbeschriebenen Ausführungsformen und Alternativen unmittelbar oder in Analogie auch für die vorbeschriebene Messvorrichtung 12 gemäß den Figuren 15 bis 17.

Die vorbeschriebenen Messvorrichtungen 12 ermöglichen sowohl eine Messung in einer aufrechten Position, wie dies in den Figuren dargestellt ist, als auch eine Überkopfmessung.

Die Durchführung einer Härtemessung einer Oberfläche des Prüfkörpers 14 mit einer Messvorrichtung 12 in einer Messanordnung 11 erfolgt folgendermaßen:
Nach dem Auflegen des Prüfkörpers 14 auf der Messtischaufnahme 26 wird oberhalb des Prüfkörpers 14 die Messvorrichtung 12 mittels des Stativs 17 positioniert. In dieser Ausgangsposition der Messvorrichtung 12 ist der Eindringkörper 41 in einer Ausgangslage, das heißt, dass der Eindringkörper 41 gegenüber einer Unterseite der Grundplatte 51 des Gehäuses 47 oder bezüglich einer Aufsetzfläche 76 am Aufsetzring 74, der am Gehäuse 47 befestigt ist, nach innen zurückversetzt ist. Anschließend wird mittels dem zumindest einen Motor 19 des Stativs 17 die Messvorrichtung 12 auf die Oberfläche des Prüfkörpers 14 zubewegt. Beim Auflegen einer Aufsetzfläche 76 des Aufsetzringes 74 der Messvorrichtung 12 auf dem Prüfkörper 14 wird die Zustellbewegung stillgesetzt. Darauffolgend wird die Krafterzeugungseinrichtung 44 aktiviert. Die Antriebseinrichtung 45 betätigt das Antriebselement 96, so dass diese eine Verfahrbewegung entlang der Verfahrachse 46 in Richtung auf den Eindringkörper 41 durchführt. Aufgrund der magnetischen Übertragungseinrichtung 66 wird der Magnetpol 67 auf den Magnetpol 68 zubewegt. Aufgrund der wirkenden abstoßenden Magnetkraft der beiden Magnetpole 67, 68 wird die Zustellbewegung entlang der Verfahrachse 46 vom Magnetpol 67 auf den Magnetpol 68 berührungsfrei übertragen. Durch die Führung 57 wird der Eindringkörper 41 entlang der Verfahrachse 46, welche vorzugsweise mit der Längsachse 43 des Übertragungselementes 42 deckungsgleich ist, nach unten in Richtung auf die Oberfläche des Prüfkörpers 14 zubewegt. Sobald der Eindringkörper 41 auf der Oberfläche des Prüfkörpers 14 aufliegt, ermittelt die erste Messeinrichtung 78 keine Änderung des Abstandes, so dass über der Steuerung 33 die Zustellbewegung der Antriebseinrichtung 45 stillgesetzt wird. Diese Ausgangslage wird als Nullposition an die Steuerung 33 weitergeleitet. Darauffolgend wird durch die Steuerung 33 eine weitere Zustellbewegung des Antriebselementes 96 angesteuert, wodurch eine Eindringbewegung des Eindringkörpers 41 in den Prüfkörper 14 angesteuert wird. Durch die erste Messeinrichtung 78 wird der Eindringweg ermittelt. Aus der Zustellbewegung des Antriebselementes 96, welches durch eine dritte Messeinrichtung 105 erfasst wird, kann die Prüfkraft ermittelt werden. Alternativ und/oder zum Abgleich kann auch mittels der vierten Messeinrichtung 110 die Prüfkraft ermittelt werden, welche auf den Eindringkörper 41 wirkt. Aus diesen Messwerten und der Kenntnis der Geometrie des Eindringkörpers 41 kann die Härte der Oberfläche des Prüfkörpers 14 ermittelt werden. Der Eindringkörper 41 kann kugelförmig oder pyramidenförmig ausgebildet sein. Bevorzugt besteht dieser aus Diamant, Topas, Korund oder Quarz.

Darauffolgend wird die Messvorrichtung 12 von dem Prüfkörper 14abgehoben und/oder das Antriebselement 96 zu einer Verfahrbewegung entgegengesetzt zum Eindringkörper 41 angesteuert. Dieses kann gleichzeitig oder aufeinanderfolgend angesteuert werden. Die Messvorrichtung 12 wird in eine Ausgangsposition zurückgeführt. Durch die Führung 47 wird der Eindringkörper 41 mit dem Übertragungsstift 42 ebenfalls in eine Ausgangslage zurückgesetzt.

Anschließend nach dem Einbringen einer Eindringstelle in den Prüfkörper 14 kann mit der optischen Erfassungseinrichtung 29 eine Abbildung der Eindringstelle ermittelt und auch eine optische Auswertung durchgeführt werden.

Zur Ermittlung der Kratzfestigkeit einer Oberfläche eines Prüfkörpers 14 wird der Prüfkörper 14 auf dem Messtisch 25 beziehungsweise einer Messtischaufnahme 26 des Messtisches 15 positioniert. Oberhalb des Prüfkörpers 14 ist die Messvorrichtung 12 positioniert, so dass ein Eindringkörper 41 durch eine Zustellbewegung senkrecht zur Oberfläche des Prüfkörpers 14 auf diesen zubewegt werden kann. Die Antriebseinrichtung 45 wird betätigt, so dass das Antriebselement 96 eine Zustellbewegung entlang der Verfahrachse 46 in Richtung auf den Eindringkörper 41 ausübt. Durch die magnetische Übertragungseinrichtung 66 wird diese Zustellbewegung in eine Verfahrbewegung des Eintrittskörpers 41 umgesetzt, so dass dieser aus einer Ausgangsposition in eine Arbeitsposition übergeführt wird. In dieser Arbeitsposition stehlt der Eindringkörper 41 gegenüber einer Unterseite der Grundplatte 51 des Gehäuses 47 beziehungsweise eines Aufsetzringes 74, der in der Ausnehmung 55 der Grundplatte 51 des Gehäuses 47 angeordnet ist, hervor.

Darauffolgend wird die Messvorrichtung 12 auf den Prüfkörper 14 zubewegt. Dies erfolgt beispielsweise durch den Motor 19. Sobald der Eindringkörper 41 auf der Oberfläche des Prüfkörpers 14 aufsetzt, wird die Zustellbewegung stillgesetzt. Dieses Aufsetzen wird durch die erste Messeinrichtung 78 erfasst. Die Messvorrichtung 12 ist in einer Startposition zum Prüfkörper 41 angeordnet. Diese Startposition wird in der Steuerung 33 als Null-Position abgespeichert. Diese Startposition kann für einen sogenannten Pre-Scan für die Ermittlung der Kratzfestigkeit vorgesehen sein. Diese Startposition kann auch für eine Messung der Oberflächenrauheit der Oberfläche des Prüfkörpers vorgesehen sein.

Ausgehend von dieser Startposition kann zunächst ein Pre-Scan durchgeführt werden, das heißt, dass die Oberfläche des Prüfkörpers 14 entlang einer vorbestimmten Verfahrstrecke des Eindringkörpers 41 abgetastet wird. Diese Verfahrstrecke ist tangential oder rechtwinklig zum Prüfkörper 14, beispielsweise entlang der X-Achse ausgerichtet. Bevorzugt steht die Messvorrichtung 12 still und der Messtisch 25 wird durch einen Motor 28 in Pfeilrichtung 27 gemäß Figur 1 verfahren, wodurch die Lage der Oberfläche und die Kontur der Oberfläche abgetastet und die Messsignale als Vorkratz-Profildaten, auch Pre-Scan genannt, abgespeichert werden. Anschließend wird die Messvorrichtung 12 von dem Prüfkörper 14 angehoben. Die Messvorrichtung 12 und der Messtisch 25 werden wieder in der Startposition positioniert. Darauffolgend wird wiederum durch die Steuerung 33 dieselbe Verfahrbewegung wie beim Pre-Scan gemäß Pfeil 27 mittels des Motors 28 angesteuert. Zeitgleich mit dieser Verfahrbewegung wird die Antriebseinrichtung 45 angesteuert, so dass der Eindringkörper 41 mit einer Prüfkraft beaufschlagt wird, wodurch der Eindringkörper 41 während der Verfahrbewegung des Messtisches 25 zunehmend in die Oberfläche des Prüfkörpers 41 eindringt. Diese Eindringbewegung wird durch die erste Messvorrichtung 48 erfasst. Gleichzeitig wird über die dritte Messvorrichtung 105 die aufgebrachte Prüfkraft errechnet. Zusätzlich kann über die vierte Messeinrichtung 110 die tatsächlich aufgebrachte Prüfkraft erfasst werden. Ergänzend wird eine Auslenkung des Eindringkörpers 41 in Verfahrrichtung gemäß Pfeil 27 mittels der zweiten Messeinrichtung 91 erfasst. Am Ende der vorbestimmten Verfahrbewegung nach Aufbringen der vorbestimmten Prüfkraft wird die Messvorrichtung 12 wiederum vom Prüfkörper 14 abgehoben. Die während dem Einbringen des Kratzers erfassten Messsignale werden durch die Steuerung 33 abgespeichert und ausgewertet, um die Kratzfestigkeit zu bestimmen.

Die Messvorrichtung 12 und der Messtisch 25 können nach dem Einbringen des Kratzers in dem Prüfkörper 14 wieder in die Startposition zurückgeführt werden. Darauffolgend kann ein sogenannter Post-Scan erfolgen. Der Eindringkörper 41 wird im Kratzer positioniert. Es erfolgt wiederum eine Verfahrbewegung des Messtisches 25 gemäß Pfeil 27, wodurch der Eindringkörper 41 entlang des Kratzers und in dem Kratzer geführt wird. Während der Verfahrbewegung des Eindringkörpers 41 in dem Kratzer werden die Messsignale wieder von der erste Messeinrichtung 78 und zumindest der zweiten Messeinrichtung 91 erfasst. Ergänzend kann während dem Pre-Scan, dem Einbringen des Kratzers und/oder dem Postscan mittels eines weiteren Sensors einer weiteren Messeinrichtung eine Auslenkung des Eindringkörpers in Y-Richtung erfasst werden, also in einer Richtung senkrecht zur X-Richtung in der Ebene der Oberfläche des Prüfkörpers 14.

Nach dem Einbringen des Kratzers und/oder dem Postscan kann die optische Erfassungseinrichtung 29 den Kratzer erfassen und ergänzend eine optische Auswertung ermöglichen.

Für die Messung der Oberflächenrauheit des Prüfkörpers 14 wird bevorzugt wie bei der Kratzfestigkeit die Startposition eingenommen. Ausgehend von dieser Startposition wird der Eindringkörper 41 entlang einer vorbestimmten Verfahrstrecke auf der Oberfläche des Prüfkörpers 14 entlang bewegt. Die Verfahrstrecke ist tangential oder rechtwinklig zum Prüfkörper 14 und beispielsweise entlang der X-Achse ausgerichtet. Dabei kann die Messvorrichtung 12 stillstehen und der Messtisch 25 wird durch einen Motor 28 in Pfeilrichtung 27 verfahren. Alternativ kann auch der Messtisch 25 stillstehen und die Messvorrichtung 12 wird verfahren. Ebenso kann eine Relativbewegung zwischen den beiden erfolgen. Die durch die Oberflächenrauheit des Prüfkörpers 14 erzeugte Verfahrbewegung des Eindringkörpers 41 entlang der Längsachse 48 wird durch die erste Messeinrichtung 78 erfasst und von der Steuerung 33 ausgewertet. Nach dem Abtasten einer vorbestimmten Verfahrstrecke entlang der Oberfläche des Prüfkörpers 14 wird die Messvorrichtung 12 wiederum von dem Prüfkörper 14 abgehoben.

## Patentansprüche

1. Messvorrichtung zur Erfassung von Messsignalen während einer Eindringbewegung eines Eindringkörpers (41) in eine Oberfläche eines Prüfkörpers (14), insbesondere zur Härtemessung, oder zur Ermittlung der Kratzfestigkeit der Oberfläche des Prüfkörpers (14) oder zur Erfassung von Messsignalen während einer Abtastbewegung des Eindringkörpers (41) auf der Oberfläche des Prüfkörpers (14), insbesondere zur Ermittlung der Oberflächenrauheit, mit einem Gehäuse (47), welches eine Krafterzeugungseinrichtung (44) aufweist, welche mit einem Eindringkörper (41) zur Erzeugung einer Verfahrbewegung des Eindringkörpers (41) entlang einer Verfahrachse (48) des Eindringkörpers (41) wirkverbunden ist und eine Eindringbewegung des Eindringkörpers (41) in die zu prüfende Oberfläche des Prüfkörpers (14) ansteuert oder den Eindringkörper (41) auf der Oberfläche des Prüfkörpers (14) zum Abtasten positioniert und mit zumindest einer ersten Messeinrichtung (78) zur Messung der Eindringtiefe in die Oberfläche des Prüfkörpers (14) oder zur Messung einer Verfahrbewegung des Eindringkörpers (41) längs dessen Verfahrachse (48) während einer Abtastbewegung des Eindringkörpers (41) auf der Oberfläche des Prüfkörpers (14), wobei
- die Krafterzeugungseinrichtung (44) eine Antriebseinrichtung (45) und eine magnetische Übertragungseinrichtung (66) aufweist, und die magnetische Übertragungseinrichtung (66) einen ersten Magnetpol und einen zweiten Magnetpol (67, 68) umfasst, die mit Abstand zueinander angeordnet sind und mit gleichen Polen zueinander ausgerichtet sind,
**dadurch gekennzeichnet,**
- **dass** der erste Magnetpol (67) mit der Antriebseinrichtung (45) verbunden ist, welche eine Verfahrbewegung des ersten Magnetpols (67) entlang einer Verfahrachse (46) ansteuert, die in der Achse der Eindringbewegung des Eindringkörpers (41) oder parallel dazu liegt oder die senkrecht zur Achse der Eindringbewegung des Eindringkörpers (41) liegt, und
- **dass** der zweite Magnetpol (68) der Übertragungseinrichtung (66) an einem Übertragungselement (42) vorgesehen ist, welches an seinem gegenüberliegenden Ende den Eindringkörper (41) aufnimmt, wobei das Übertragungselement (42) entlang einer Verfahrachse (48) verfahrbar im Gehäuse (47) geführt ist, welche senkrecht zu einer Grundplatte (51) des Gehäuses (47) ist oder in der Achse der Eindringbewegung des Eindringkörpers (41) liegt,
- und eine durch die Antriebseinrichtung (45) angesteuerte Verfahrbewegung mittels der Magnetkraft der magnetischen Übertragungseinrichtung (66) auf den Eindringkörper (41) übertragen wird.

2. Messvorrichtung nach Anspruch 1, **dadurch gekennzeichnet, dass** durch die Verfahrbewegung des ersten Magnetpols (67) in Richtung auf den zweiten Magnetpol (68) die Verfahrbewegung des Eindringkörpers (41) in Richtung auf den Prüfkörper für eine Eindringkraft in den Prüfkörper (14) oder eine Auflagekraft auf dem Prüfkörper (14) zum Abtasten der Oberfläche des Prüfkörpers (14) einstellbar ist.

3. Messvorrichtung nach einem der Ansprüche 1 oder 2, **dadurch gekennzeichnet, dass** das Übertragungselement (42) durch eine an einer Haltevorrichtung (58) angeordnete Führung (57) im Gehäuse (47) verfahrbar aufgenommen ist und die Führung (57) zumindest zwei zueinander beabstandete, nachgiebige Elemente, vorzugsweise zwei parallel zueinander beabstandete Blattfederelemente (61, 62) oder zwei parallel zueinander beabstandete Druckmembranelemente (151, 152), aufweist, die das Übertragungselement (42) in der Verfahrachse (46) der Antriebseinrichtung (45) verfahrbar führen, und die Führung (57) in der Haltevorrichtung (58) lösbar befestigt, insbesondere geklemmt, gehalten ist oder die Führung (57) einstückig mit der Haltevorrichtung (58) verbunden ist, wobei vorzugsweise die Haltevorrichtung (58) und die einstückig daran angeordneten Blattfederelemente (61, 62) durch Erodieren oder Feinstbearbeitung hergestellt sind.

4. Messvorrichtung nach einem der vorhergehenden Ansprüche, **dadurch gekennzeichnet, dass** das Gehäuse (47) eine Grundplatte (51) mit einer Ausnehmung (55) umfasst, und die Verfahrbewegung des Eindringkörpers (41) zur Längsachse der Ausnehmung (55) ausgerichtet ist und der Eindringkörper (41), welcher am unteren Ende des Übertragungselementes (42) vorgesehen ist, aus einer Ausgangsposition innerhalb der Ausnehmung (55) oder innerhalb eines in der Ausnehmung (55) angeordneten Aufsetzringes (75) gegenüber einer Außenseite der Grundplatte (51) hervorstehende Antriebsposition positionierbar ist, und vorzugsweise die Führung (57) das Übertragungselement (42) mit dem daran angeordneten Eindringkörper (41) in einer Ausgangsposition hält, in der der Eindringkörper (41) gegenüber einer Unterseite des Gehäuses (47), welche zum Prüfkörper (14) ausgerichtet ist, nach innen zurückversetzt angeordnet ist.

5. Messvorrichtung nach Anspruch 4, **dadurch gekennzeichnet, dass** an der Grundplatte (51) des Gehäuses (47) benachbart zum Eindringkörper (41) die erste Messeinrichtung (78) vorgesehen ist, welche vorzugsweise einen Messfühler (77) aufweist, der an einem dem Eindringkörper (41) innenliegenden Ende zugeordnet ist.

6. Messvorrichtung nach einem der vorhergehenden Ansprüche, **dadurch gekennzeichnet, dass** die Antriebseinrichtung (45) an einem Deckelelement (52) des Gehäuses (47) vorgesehen ist, welche zumindest ein entlang der Verfahrachse (46) verfahrbares Antriebselement (96) aufweist, welche in der Verfahrachse (48) des Eindringkörpers (41) oder parallel zur Verfahrachse (48) zum Eindringkörper (41) liegt und das Antriebselement (96) an einem zum Übertragungselement (42) weisenden Ende den ersten Magnetpol (67) aufnimmt, und vorzugsweise das Antriebselement (96) als Antriebsspindel mit einer am Gehäuse (47) vorgesehenen Führung (106) verdrehsicher, insbesondere einer am Deckelelement (52) angeordneten Führung (106), geführt ist, oder dass das Antriebselement (96) als eine Teleskopspindel ausgebildet und dass das Antriebselement (96) mit einem Drehantrieb (98), der vorzugsweise als Zahnriemenantrieb ausgebildet ist und das Antriebselement (96) antreibt, verbunden ist, welcher von einem Antriebsmotor (97) angetrieben ist.

7. Messvorrichtung nach einem der Ansprüche 1 bis 5, **dadurch gekennzeichnet, dass** die Verfahrachse (46) des Antriebselements (96) senkrecht zur Verfahrachse (48) des Eindringkörpers (41) ausgerichtet ist und das Antriebselement (96) eine Verfahrbewegung des zumindest einen ersten Magnetpols (67), vorzugsweise eine gleichzeitige Verfahrbewegung von zwei oder mehreren den ersten Magnetpol (67) bildenden Permanentmagnete, ansteuert, welche in eine teilweise überdeckende Position oder in eine deckungsgleiche Position zu einer entsprechenden Anzahl von Permanentmagneten, die den zweiten Magnetpol (68) bilden, überführbar sind, und das Übertragungselement (42) eine Aufnahmeeinrichtung (71) aufweist, welche in gleichem Abstand zur Verfahrachse (48) des Übertragungselementes (42) zumindest zwei Permanentmagnete zur Bildung des zweiten Magnetpols (68) aufnimmt und vorzugsweise die Permanentmagnete des ersten Magnetpols (67) gleichzeitig in Richtung auf die Permanentmagnete des zweiten Magnetpols (68) zur teilweisen Überdeckung oder deckungsgleichen Anordnung verfahrbar sind.

8. Messvorrichtung nach Anspruch 7, **dadurch gekennzeichnet, dass** das Antriebselement (96) aus einem Paar Zahnstangen (161, 162) gebildet ist, welches mit einem Drehantrieb (98) senkrecht zur Verfahrachse (48) des Eindringkörpers (41) ansteuerbar und vorzugsweise entlang von Führungsschienen (166) verfahrbar sind und insbesondere auf die gegenüberliegende Zahnstange (161, 162) zuweisend jeweils ein Permanentmagnet zur Bildung des ersten Magnetpols (67) vorgesehen ist.

9. Messvorrichtung nach einem der Ansprüche 6 bis 8, **dadurch gekennzeichnet, dass** eine Antriebsbewegung des Antriebselementes (96) mit einer dritten Messeinrichtung (105) überwacht ist, und vorzugsweise zwischen dem Antriebselement (96) und dem daran angeordneten ersten Magnetpol (67) eine vierte Messeinrichtung (110), insbesondere ein Kraftsensor, vorgesehen ist.

10. Messvorrichtung nach einem der vorhergehenden Ansprüche, **dadurch gekennzeichnet, dass** dem am Übertragungselement (42) angeordneten zweiten Magnetpol (68) eine Schwingungsdämpfungseinrichtung (120) zugeordnet ist, welches vorzugsweise ein aus einem ferromagnetischen Material hergestellte Umhausung (121), insbesondere Rohr, ausgebildet ist, welche den zweiten Magnetpol (68) umgibt und in einer Ausgangsposition des Eindringkörpers (41) der zweite Magnetpol (68) zumindest teilweise in die Umhausung (121) eingetaucht ist.

11. Messvorrichtung nach einem der vorhergehenden Ansprüche, **dadurch gekennzeichnet, dass** zwischen den zwei parallel zueinander beabstandeten Blattfederelementen (61, 62) ein Ausgleichselement (89) vorgesehen ist, welches an der Haltevorrichtung (58) schwenkbar gelagert ist und vorzugsweise das Ausgleichselement (89) mit einem Ende in das Übertragungselement (42) ragt, an welchem ein Blattfederelement (94) vorgesehen ist, welches sich in Richtung auf ein den Magnetpol (68) aufnehmendes Ende des Übertragungselementes (42) erstreckt und daran befestigt ist, und vorzugsweise das Ausgleichselement (89) mittels einer Spanneinrichtung (85), insbesondere einer Spannbandlagerung, an der Haltevorrichtung (58) gelagert ist.

12. Messanordnung zum Erfassen einer Eindringtiefe in einer Oberfläche eines Prüfkörpers (14), insbesondere zur Erfassung der Kratzfestigkeit einer Oberfläche eines Prüfkörpers (14), oder zum Erfassen einer Oberflächenrauheit einer Oberfläche eines Prüfkörpers (14),
- mit einer Messvorrichtung (12) nach einem der Ansprüche 1 bis 11,
- mit einem Messtisch (25) zur Aufnahme des Prüfkörpers (14),
- mit einer Handhabungseinrichtung (17) zum Überführen einer Messvorrichtung (12) aus einer Ausgangsposition (21) in eine Messposition (22),
- mit einem Grundkörper (16), auf dem zumindest der Messtisch (25) und die Handhabungseinrichtung (17) vorgesehen sind,
- mit einer Steuerung (33) zur Ansteuerung und Durchführung einer Messung mit der Messvorrichtung (12) an dem Prüfkörper (14), welche zumindest ein Aufsetzen eines Eindringkörpers (41) der Messvorrichtung (12) auf dem Prüfkörper (14) mit der Handhabungsvorrichtung (17) ansteuert, wobei durch die Messvorrichtung (12) die Eindringbewegung des Eindringkörpers (41) in die Oberfläche des Prüfkörpers (14) oder die Abtastbewegung des Eindringkörpers (41) auf der Oberfläche des Prüfkörpers (14) angesteuert ist.

13. Messanordnung nach Anspruch 12, **dadurch gekennzeichnet, dass** die optische Erfassungseinrichtung (29) benachbart zur Messvorrichtung (12) auf dem Grundkörper (16) angeordnet ist, wobei der Messtisch (25) zwischen der Messvorrichtung (12) und der optischen Erfassungseinrichtung (29) verfahrbar oder die Messvorrichtung (12) und die optische Erfassungseinrichtung (29) zum Messtisch (25) verfahrbar sind und vorzugsweise eine Verfahrbewegung des Messtisches (25), insbesondere entlang einer in der Ebene der Oberfläche des Prüfkörpers (14) liegenden Achse, durch die Steuerung (33) angesteuert ist.

14. Verfahren zum Erfassen von Messsignalen während einer Eindringbewegung eines Eindringkörpers (41) in eine Oberfläche eines Prüfkörpers (14) einer Messvorrichtung (12), oder während einer Abtastbewegung eines Eindringkörpers (41) auf einer Oberfläche eines Prüfkörpers (14), bei dem der Prüfkörper (14) auf einem Messtisch (25) positioniert und die Messvorrichtung (12) auf den Prüfkörper (14) in einer Startposition aufgesetzt wird, bei dem die Eindringbewegung oder die Abtastbewegung des Eindringkörpers (41) mit der Krafterzeugungseinrichtung (44) angesteuert wird, welche eine Antriebseinrichtung (45) und eine magnetische Übertragungseinrichtung (45) umfasst, wobei die magnetische Übertragungseinrichtung (66) einen ersten Magnetpol und einen zweiten Magnetpol (67, 68) umfasst, die mit Abstand zueinander angeordnet sind und mit gleichen Polen zueinander ausgerichtet sind, bei dem durch eine Zustellbewegung der Antriebseinrichtung (43) mit der Magnetkraft die Eindringbewegung des Eindringkörpers (41) in den Prüfkörper (14) oder bei der mit einer Magnetkraft die Abtastbewegung des Eindringkörpers (41) auf dem Prüfkörper (14) angesteuert wird.

15. Verfahren nach Anspruch 14, **dadurch gekennzeichnet, dass** zur Härtemessung der Oberfläche des Prüfkörpers (14) in einem ersten Verfahrensschritt die Messvorrichtung (12) auf den Prüfkörper (14) zubewegt wird, dass beim Aufsetzen der Messvorrichtung (12) auf dem Prüfkörper (14) die Zustellbewegung stillgesetzt wird, dass eine Verfahrbewegung des Eindringkörpers (41) angesteuert wird, bis dieser auf der Oberfläche des Prüfkörpers (14) aufliegt und diese Position als Nullposition für die nachfolgende Härtemessung an die Steuerung (33) weitergeleitet wird oder dass zur Kratzfestigkeitsmessung der Oberfläche des Prüfkörpers (14) in einem ersten Verfahrensschritt vor dem Aufsetzen der Messvorrichtung (12) auf die Oberfläche des Prüfkörpers (14) der Eindringkörper (41) mit einer Verfahrbewegung beaufschlagt wird, so dass der Eindringkörper (41) gegenüber einer Unterseite des Gehäuses (47) frei hervorsteht, dass die Messvorrichtung (12) auf den Prüfkörper (14) zubewegt wird und beim Aufsetzen des Eindringkörpers (41) auf dem Prüfkörper (14) stillgesetzt wird und diese Position als Nullposition für die nachfolgende Kratzfestigkeitsmessung an die Steuerung (33) weitergeleitet wird, und dass während der mit der Magnetkraft angesteuerten Eindringbewegung des Eindringkörpers (41) in dem Prüfkörper (14) der Messtisch (25) in einer Richtung senkrecht zur Eindringbewegung des Eindringkörpers (41) verfahren und ein Kratzer in die Oberfläche des Prüfkörpers (14) eingebracht wird und die Messsignale der ersten Messeinrichtung (78) für die Eindringtiefe und die Messsignale zumindest einer weiteren, dem Eindringkörper (41) zugeordneten zweiten Messeinrichtung (91) eine Auslenkung des Eindringkörpers (41) entlang der Verfahrrichtung des Prüfkörpers (14) sowie die errechnete Kraft durch die erfasste Zustellbewegung des Antriebelementes (96) mit der dritten Messeinrichtung (105) oder die erfassten Messsignale der vierten Messeinrichtung (110) erfasst und ausgewertet werden.

16. Verfahren nach Anspruch 14 bis 15, **dadurch gekennzeichnet, dass** die Krafterzeugungseinrichtung (44) mit einer Prüfkraft beaufschlagt wird und eine Eindringbewegung des Eindringkörpers (41) in die Oberfläche des Prüfkörpers (14) zumindest mit einer ersten Messeinrichtung (78) erfasst wird, und vorzugsweise dass die auf den Eindringkörper (41) wirkende Kraft aus der Zustellbewegung, die durch eine dritte Messeinrichtung (105) erfasst wird, errechnet oder durch eine vierte Messeinrichtung (110) erfasst wird und dass durch die erste Messeinrichtung (78) die Eindringtiefe des Eindringkörpers (41) in den Prüfkörper (14) erfasst und dass aus der errechneten oder der erfassten Eindringkraft durch die dritte oder vierte Messeinrichtung (105, 110) und der erfassten Eindringtiefe durch die erste Messeinrichtung (78) in Abhängigkeit der Geometrie des Eindringkörpers (41) die Härte der Oberfläche des Prüfkörpers (14) bestimmt wird.

17. Verfahren nach einem der Ansprüche 14 bis 16, **dadurch gekennzeichnet, dass** vor dem Einbringen des Kratzers in den Prüfkörper (14) die Messvorrichtung (12) auf der Oberfläche des Prüfkörpers (14) aufgesetzt und in einer Richtung senkrecht zur Aufsetzbewegung des Prüfkörpers (14) verfahren wird und die durch die erste Messeinrichtung (78) erfassten Messsignale erfasst und als Vorkratz-Profildaten gespeichert werden und/oder dass nach dem Einbringen des Kratzers in den Prüfkörper (14) die Messvorrichtung (12) in dem Kratzer aufgesetzt und der Eindringkörper (41) mit der Messvorrichtung (12) in einer Richtung senkrecht zur Aufsetzbewegung des Prüfkörpers (14) verfahren wird und die durch die Messvorrichtung (12) erfassten Signale entlang der Verfahrbewegung des Eindringkörpers (41) in dem Kratzer erfasst und als Nachkratz-Profildaten gespeichert werden.

## Claims

1. A measuring device for detecting measuring signals during a penetration movement of an indenter (41) into a surface of a specimen (14), in particular for measuring the hardness, or for determining the scratch resistance of the surface of the specimen (14) or for detecting measuring signals during a scanning movement of the indenter (41) on the surface of the specimen (14), in particular for determining the surface roughness, with a housing (47) comprising a force-generation means (44) which is operatively-connected with an indenter (41) for generating a displacing movement of the indenter (41) along a travel axis (48) of the indenter (41), and which drives a penetrating movement of the indenter (41) into the surface to be tested of the specimen (14) or which positions the indenter (41) on the surface of the specimen (14) for scanning, and with at least one first measuring means (78) for measuring the penetration depth into the surface of the specimen (14) or for measuring a displacing movement of the indenter (41) along its travel axis (48) during a scanning movement of the intender (41) on the surface of the specimen (14), wherein
- the force generation means (44) comprises a drive device (45) and a magnetic transmission device (66),
- the magnetic transmission device (66) comprises a first magnetic pole and a second magnetic pole (67, 68), which are arranged at a distance to one another and which are oriented with the same poles to one other,
**characterized in**
- **that** the first magnetic pole (67) is connected with the drive device (45) that drives a displacing movement of the first magnetic pole (67) along a travel axis (46), which is located in the axis of the penetration movement of the indenter (41) or parallel thereto, or which is located perpendicular to the axis of the penetration movement of the indenter (41), and
- **that** the second magnetic pole (68) of the transmission device (66) is provided on a transmission element (42) which receives the indenter (41) on the opposite end thereof, wherein the transmission element (42) is displaceably guided inside the housing (47) along a travel axis (48), which axis preferably is perpendicular to a base plate (51) of the housing (47) or is located in the axis of the penetrating movement of the indenter (41),
- and transmits a travel movement driven by the drive device (65) on to the indenter (41) by means of a magnetic force of the magnetic transmission device (66).

2. The measuring device according to claim 1, **characterized in that** by the displacing movement of the first magnetic pole (67) in the direction towards the second magnetic pole (68), the displacing movement of the indenter (41) in the direction towards the specimen for a penetration force into the specimen (14) or a contact force on the specimen (14) for scanning the surface of the specimen (14) is adjustable.

3. The measuring device according to one of claims 1 or 2, **characterized in that** the transmission element (42) is displaceably accommodated in the housing (47) by means of a guide (57) arranged on a holding device (58), and the guide (57) comprises at least two resilient elements spaced from one another, preferably two leaf-spring elements (61, 62) parallelly spaced from one another, or two pressure diaphragm elements (151, 152) parallelly spaced from one another (151, 152), which displaceably guide the transmission element (42) in the travel axis (46) of the drive device (45), and the guide (57) is releasably held in the holding device (58), in particular clamped, or that the guide (57) is integrally connected with the holding device (58), wherein the holding device (58) and the leaf-spring elements (61, 62) integrally arranged thereon are preferably produced by means of eroding or ultrafine processing.

4. The measuring device according to any one of the preceding claims, **characterized in that** the housing (47) comprises a base plate (51) with a recess (55), and the displacing movement of the indenter (41) is aligned with the longitudinal axis of the recess (55), and the indenter (41), which is provided at the lower end of the transmission element (42), can be positioned, from an initial position inside the recess (55) or inside an attachment ring (75) arranged in the recess (55), with respect to an outer side of the base plate (51) in a drive position protruding with respect to an outer side of the base plate (51), and preferably the guide (57) holds the transmission element (42) with the indenter (41) arranged thereon in an initial position, in which the indenter (41) is arranged set-back inwardly with respect to a lower side of the housing (47) that is oriented towards the specimen (14).

5. The measuring device according to claim 4, **characterized in that** the first measuring means (78) is provided on the baseplate (51) of the housing (47) adjacent to the indenter (41), which means preferably comprises a measuring probe (77) which is assigned to an internal end of the indenter (41).

6. The measuring device according to any one of the preceding claims, **characterized in that** the drive device (45) is provided on a cover element (52) of the housing (47), which comprises at least one drive element (96) displaceable along the travel axis (46), which is located in the travel axis (48) of the indenter (41) or in parallel to the travel axis (48) of the indenter (41), and the drive element (96) receives the first magnetic pole (67) at an end directed towards the transmission element (42), and preferably the drive element (96) is guided, as a drive spindle with a guide (106) provided on the housing (47), in a manner secured against rotation, in particular with a guide (106) arranged on the cover element (52), or that the drive element (96) is configured as a telescopic spindle and that the drive element (96) is connected with a rotary drive (98), which preferably is configured as a belt drive and drives the drive element (96), which is driven by the drive motor (97).

7. The measuring device according to any one of claims 1 to 5, **characterized in that** the travel axis (46) of the drive element (96) is oriented perpendicular to the travel axis (48) of the indenter (41), and the drive element (96) drives a displacing movement of the at least one first magnetic pole (67), preferably a simultaneous displacing movement of two or more permanent magnets forming the first magnetic pole (67), which can be transferred to a partially overlapping position or to a congruent position with respect to a corresponding number of permanent magnets forming the second magnetic pole (68), and the transmission element (42) comprises a receiving device (71), which, at the same distance to the travel axis (48) of the transmission element (42), receives at least two permanent magnets for forming the second magnetic pole (68), and preferably the permanent magnets of the first magnetic pole (67) are at the same time displaceable in the direction towards the permanent magnets of the second magnetic pole (68) for a partial overlap or congruent arrangement.

8. The measuring device according to claim 7, **characterized in that** the drive element (96) is formed by a pair of toothed racks (161, 162), which is actuatable with a rotary drive (98) perpendicular to the travel axis (48) of the indenter (41) and which is preferably displaceable along guide rails (166), and in particular respectively one permanent magnet for forming the first magnetic pole (67) is provided in a manner facing the opposite toothed rack (161, 162).

9. The measuring device according to any one of claims 6 to 8, **characterized in that** a drive movement of the drive element (96) is monitored by a third measuring means (105), and preferably a fourth measuring means (110), in particular a force sensor, is provided between the drive element (96) and the first magnetic pole (67) arranged thereon.

10. The measuring device according to any one of the preceding claims, **characterized in that** a vibration damping device (120) is assigned to the second magnetic pole (68) arranged on the transmission element (42), which preferably is formed as an enclosure (121) made of a ferromagnetic material, in particular a tube, which surrounds the second magnetic pole (68) and, in an initial position of the indenter (41), the second magnetic pole (68) is at least partially plunged in the enclosure (121).

11. The measuring device according to any one of the preceding claims, **characterized in that** a compensating element (89) is provided between the two leaf spring elements (61, 62) spaced in parallel from one another, which element is pivotably mounted on the holding device (58) and preferably the compensating element (89) protrudes into the transmission element (42) with an end thereof, on which a leaf spring element (94) is provided that extends in the direction towards an end of the transmission element (42) that receives the magnetic pole and is fixed thereto, and preferably the compensating element (89) is mounted on the holding device (58) by means of a clamping means (85), in particular a strap mounting.

12. The measuring arrangement for detecting a penetration depth in a surface of a specimen (14), in particular for detecting the scratch resistance of a surface of a specimen (14), or for detecting a surface roughness of a surface of a specimen (14), comprising
- a measuring device (12) according to one of the claims 1 to 11,
- a measuring table (25) for receiving the specimen (14),
- a handling means (17) for transferring a measuring device (22) from an initial position into a measuring position (22),
- a base body (16), on which at least the measuring table (25) and the handling means (17) is provided,
- a control (33) for driving and performing a measurement with the measuring device (12) on the specimen (14), which drives at least a placing movement of an indenter (41) of the measuring device (12) on to the specimen (14) with the handling device (17), that the penetration movement of the indenter (41) into the surface of the specimen (14) or the scanning movement of the indenter (41) on the surface of the specimen (14) is controlled by the measuring device (12).

13. The measuring arrangement according to claim 12, **characterized in that** the optical detection means (29) is arranged adjacent to the measuring device (12) on the base body (16), wherein the measuring table (25) is displaceable between the measuring device (12) and the optical detection means (29), or the measuring device (12) and the optical detection means (29) are displaceable relative to the measuring table (25), and particularly a displacing movement of the measuring table (25), in particular along an axis located in the plane of the surface of the specimen (14), is driven by the control (33).

14. A method for detecting measuring signals during a penetration movement of an indenter (41) into a surface of a specimen (14) of a measuring device (12), or during a scanning movement of an indenter (41) on a surface of a specimen (14), in which the specimen (14) is positioned on a measuring table (25) and the measuring device (12) is placed on to the specimen (14), in an initial position, the penetrating movement or the scanning movement of the indenter (41) is driven with the force generation means (44), which comprises a drive device (45) and a magnetic transmission device, wherein the magnetic transmission device (66) comprises a first magnetic pole and a second magnetic pole (67, 68), which are arranged at a distance to one another and which are oriented with the same poles to one other, wherein by a feed movement of the drive device (43) using a magnetic force, the penetrating movement of the indenter (41) into the specimen (14) is driven, or using a magnetic force, the scanning movement of the indenter (41) on the specimen (14) is driven.

15. The method according to claim 14, **characterized in that** for measuring the hardness of the surface of the specimen (14), in a first method step, the measuring device (12) is moved towards the specimen (14), that when placing the measuring device (12) on to the specimen (14), the feed movement is immobilized, that a displacing movement of the indenter (41) is driven until the indenter rests on the surface of the specimen (14), and this position is forwarded to the control (33) as a zero position for the subsequent hardness measurement, or that for scratch-resistance measurement of the surface of the specimen (14), in a first method step, prior to the measuring device (12) being placed on to the surface of the specimen (14), the indenter (41) is applied with a displacing movement, so that the indenter (41) freely protrudes with respect to a lower side of the housing (47), that the measuring device (12) is moved towards the specimen (14) and is immobilized when the indenter (41) is placed on to the specimen (41), and this position is forwarded to the control (33) as a zero position for the subsequent scratch resistance measuring, and that during the penetration movement of the indenter (41) in the specimen (14), driven by the magnetic force, the measuring table (25) is displaced in a direction perpendicular to the penetrating movement of the indenter (41) and a scratch is introduced into the surface of the specimen (14), and via the measuring signals of the first measuring means (78) for the penetration depth and the measuring signals of at least one further, second measuring means (91) assigned to the indenter (41), a deflection of the indenter (41) along the displacement direction of the specimen (41) as well as the calculated force by the detected feed movement of the drive element (96) with the third measuring means (105) or the detected measuring signals of the fourth measuring means (110) are detected and evaluated.

16. The method according to claim 14 to 15, **characterized in that** the force generation means (44) is applied with a testing force and that a penetrating movement of the indenter (41) into the surface of the specimen (41) is detected at least with a first measuring means (78), and preferably that the force from the feed movement that acts on the indenter (41), which is detected by third measuring means (105), is calculated, or detected by a fourth measuring means (110), and that the penetration depth of the indenter (41) into the specimen (14) is detected by the first measuring means (78), and that the hardness of the surface of the specimen (14) is determined from the calculated or detected penetration force by the third or fourth measuring device (105, 110) and the detected penetration depth through the first measuring means (78) depending on the geometry of the indenter (41).

17. The method according to any one of claims 14 to 16, **characterized in that** prior to the introduction of the scratch in the specimen (14), the measuring device (12) is placed on to the surface of the specimen (14) and is displaced in a direction perpendicular to the place-on movement of the specimen (14), and the measuring signals detected by the first measuring means (78) are detected and stored as pre-scratch profile data and/or that the after the introduction of the scratch into the specimen (14), the measuring device (12) is placed in the scratch, and the intender (41) with the measuring device (12) is displaced in a direction perpendicular to the place-on movement of the specimen (14), and the signals detected by the measuring device (12) are detected along the displacing movement of the indenter (41) in the scratch, and are stored as post-scratch profile data.

## Revendications

1. Dispositif de mesure destiné à saisir des signaux de mesure pendant un mouvement de pénétration d'un corps de pénétration (41) dans une surface d'un corps testé (14), en particulier en vue de mesurer la dureté ou de déterminer la résistance aux rayures de la surface du corps testé (14), ou destiné à saisir des signaux de mesure pendant un mouvement de balayage du corps de pénétration (41) sur la surface du corps testé (14), en particulier en vue de déterminer la rugosité de surface, pourvu d'un boîtier (47) qui présente un dispositif générateur de force (44) qui est relié de manière opérationnelle à un corps de pénétration (41) en vue de générer un mouvement de déplacement du corps de pénétration (41) le long d'un axe de déplacement (48) du corps de pénétration (41) et qui active un mouvement de pénétration du corps de pénétration (41) dans la surface à tester du corps testé (14), ou qui positionne le corps de pénétration (41) sur la surface du corps testé (14) en vue de procéder au balayage, et pourvu d'au moins un premier organe de mesure (78) destiné à mesurer la profondeur de pénétration dans la surface du corps testé (14) ou destiné à mesurer un mouvement de déplacement du corps de pénétration (41) le long de son axe de déplacement (48) pendant un mouvement de balayage du corps de pénétration (41) sur la surface du corps testé (14),
- le dispositif générateur de force (44) présentant un dispositif d'entraînement (45) et un dispositif de transmission magnétique (66) et le dispositif de transmission magnétique (66) comprenant un premier pôle magnétique et un deuxième pôle magnétique (67, 68) qui sont disposés de manière espacée entre eux et dont les pôles identiques sont orientés l'un vers l'autre,
**caractérisé en ce que**
- le premier pôle magnétique (67) est relié au dispositif d'entraînement (45) qui active un mouvement de déplacement du premier pôle magnétique (67) le long d'un axe de déplacement (46) qui se situe dans l'axe du mouvement de pénétration du corps de pénétration (41) ou parallèlement à celui-ci, ou qui se situe perpendiculairement à l'axe du mouvement de pénétration du corps de pénétration (41), et- le deuxième pôle magnétique (68) du dispositif de transmission (66) est prévu sur un élément de transmission (42) qui reçoit, à son extrémité opposé, le corps de pénétration (41), l'élément de transmission (42) étant guidé de manière à pouvoir se déplacer dans le boîtier (47) le long d'un axe de déplacement (48) qui est perpendiculaire à une plaque de base (51) du boîtier (47) ou qui se situe dans l'axe du mouvement de pénétration du corps de pénétration (41), et
- un mouvement de déplacement activé par le dispositif d'entraînement (45) est transmis au corps de pénétration (41) au moyen de la force magnétique du dispositif de transmission magnétique (66).

2. Dispositif de mesure selon la revendication 1, **caractérisé en ce que** le mouvement de déplacement du corps de pénétration (41) en direction du corps testé peut être réglé par le mouvement de déplacement du premier pôle magnétique (67) en direction du deuxième pôle magnétique (68) afin d'obtenir une force de pénétration donnée dans le corps testé (14) ou une force d'appui donnée sur le corps testé (14) en vue de balayer la surface du corps testé (14).

3. Dispositif de mesure selon l'une quelconque des revendications 1 ou 2, **caractérisé en ce que** l'élément de transmission (42) est reçu par un guide (57) disposé sur un dispositif de retenue (58) de manière à pouvoir se déplacer dans le boîtier (47) et **en ce que** le guide (57) présente au moins deux éléments souples espacés entre eux, de préférence deux éléments à ressort à lame (61, 62) espacés parallèlement entre eux ou deux éléments à membrane de pression (151, 152) espacés parallèlement entre eux qui guident l'élément de transmission (42) de manière à ce qu'il puisse se déplacer dans l'axe de déplacement (46) du dispositif d'entraînement (45), et **en ce que** le guide (57) est maintenu fixé de manière amovible, en particulier par serrage, dans le dispositif de retenue (58) ou **en ce que** le guide (57) est relié au dispositif de retenue (58) de manière à former une seule pièce, le dispositif de retenue (58) et les éléments à ressort à lame (61, 62) disposés sur celui-ci de manière à former une seule pièce étant fabriqués de préférence par électroérosion ou par micro-usinage.

4. Dispositif de mesure selon l'une quelconque des revendications précédentes, **caractérisé en ce que** le boîtier (47) comprend une plaque de base (51) pourvue d'un évidement (55) et **en ce que** le mouvement de déplacement du corps de pénétration (41) est orienté par rapport à l'axe longitudinal de l'évidement (55) et **en ce que** le corps de pénétration (41) qui est prévu à l'extrémité inférieure de l'élément de transmission (42) peut être positionné, à partir d'une position initiale à l'intérieur de l'évidement (55) ou à l'intérieur d'un anneau de pose (75) disposé dans ledit évidement (55), dans une position d'entraînement qui fait saillie par rapport à une face extérieure de la plaque de fond (51), et **en ce que** de préférence le guide (57) maintient l'élément de transmission (42) ainsi que le corps de pénétration (41) disposé sur ce dernier dans une position initiale dans laquelle le corps de pénétration (41) se trouve décalé vers l'intérieur par rapport à une face inférieure du boîtier (47) qui est orientée vers le corps testé (14).

5. Dispositif de mesure selon la revendication 4, **caractérisé en ce que** sur la plaque de fond (51) du boîtier (47) est prévu, de manière contiguë au corps de pénétration (41), le premier organe de mesure (78) qui présente de préférence un capteur de mesure (77) qui est affecté à une extrémité du corps de pénétration (41) située à l'intérieur.

6. Dispositif de mesure selon l'une quelconque des revendications précédentes, **caractérisé en ce que** le dispositif d'entraînement (45) est prévu sur un élément de couverture (52) du boîtier (47) et présente au moins un élément d'entraînement (96) pouvant être déplacé le long de l'axe de déplacement (46) qui se situe dans l'axe de déplacement (48) du corps de pénétration (41) ou parallèlement audit axe de déplacement (48) du corps de pénétration (41), et **en ce que** l'élément d'entraînement (96) reçoit le premier pôle magnétique (67) à une extrémité tournée vers l'élément de transmission (42), et que de préférence l'élément d'entraînement (96) est réalisé en tant que broche d'entraînement et est guidé sans pouvoir tourner par un guide (106) prévu sur le boîtier (47), en particulier par un guide (106) disposé sur l'élément de couverture (52), ou **en ce que** l'élément d'entraînement (96) est réalisé en tant que broche télescopique et que l'élément d'entraînement (96) est relié à un système d'entraînement en rotation (98), réalisé de préférence en tant qu'entraînement par courroie dentée, lequel entraîne l'élément d'entraînement (96) et est entraîné par un moteur d'entraînement (97) .

7. Dispositif de mesure selon l'une quelconque des revendications 1 à 5, **caractérisé en ce que** l'axe de déplacement (46) de l'élément d'entraînement (96) est orienté de manière perpendiculaire à l'axe de déplacement (48) du corps de pénétration (41) et que l'élément d'entraînement (96) active un mouvement de déplacement dudit au moins un premier pôle magnétique (67), de préférence un mouvement de déplacement simultané de deux ou plusieurs aimants permanents qui forment le premier pôle magnétique (67) et peuvent passer dans une position recouvrant partiellement ou une position recouvrant entièrement un nombre correspondant d'aimants permanents qui forment le deuxième pôle magnétique (68), et que l'élément de transmission (42) présente un dispositif de réception (71) qui reçoit au moins deux aimants permanents espacés de manière identique par rapport à l'axe de déplacement (48) de l'élément de transmission (42) en vue de former le deuxième pôle magnétique (68) et **en ce que** de préférence les aimants permanents du premier pôle magnétique (67) peuvent être déplacés de manière simultanée en direction des aimants permanents du deuxième pôle magnétique (68) de manière à former une disposition qui coïncide partiellement ou entièrement.

8. Dispositif de mesure selon la revendication 7, **caractérisé en ce que** l'élément d'entraînement (96) est formé par une paire de crémaillères (161, 162) lesquelles peuvent être activées perpendiculairement à l'axe de déplacement (48) du corps de pénétration (41) par un système d'entraînement en rotation (98) et qui peuvent être déplacées de préférence le long de rails de guidage (166), et sur lesquelles est prévu respectivement un aimant permanent qui est en particulier tourné respectivement vers la crémaillère opposée (161, 162) en vue de former le premier pôle magnétique (67) .

9. Dispositif de mesure selon l'une quelconque des revendications 6 à 8, **caractérisé en ce qu'**un mouvement d'entraînement de l'élément d'entraînement (96) est surveillé par un troisième organe de mesure (105) et **en ce qu'**un quatrième organe de mesure (110), en particulier un capteur de force, est prévu de préférence entre l'élément d'entraînement (96) et le premier pôle magnétique (67) disposé sur celui-ci.

10. Dispositif de mesure selon l'une quelconque des revendications précédentes, **caractérisé en ce que** le deuxième pôle magnétique (68) disposé sur l'élément de transmission (42) se voit affecter un dispositif amortisseur de vibrations (120) qui est formé de préférence par une enceinte (121) fabriquée en un matériau ferromagnétique, en particulier un tube, qui entoure le deuxième pôle magnétique (68) et **en ce que**, lorsque le corps de pénétration (41) se trouve dans une position initiale, le deuxième pôle magnétique (68) est plongé au moins partiellement dans l'enceinte (121).

11. Dispositif de mesure selon l'une quelconque des revendications précédentes, **caractérisé en ce qu'**entre les deux éléments à ressort à lame (61, 62) espacés parallèlement entre eux est prévu un élément de compensation (89) qui est monté sur le dispositif de retenue (58) de manière à pouvoir pivoter, et **en ce que** de préférence l'élément de compensation (89) pénètre avec une extrémité dans l'élément de transmission (42) sur lequel est prévu un élément à ressort à lame (94) qui s'étend en direction d'une extrémité de l'élément de transmission (42) recevant le pôle magnétique (68) et qui est fixé à celle-ci et **en ce que** de préférence l'élément de compensation (89) est monté sur le dispositif de retenue (58) au moyen d'un dispositif de serrage (85) en particulier d'un palier tendeur.

12. Système de mesure destiné à saisir une profondeur de pénétration dans une surface d'un corps testé (14), en particulier destiné à saisir la résistance aux rayures d'une surface d'un corps testé (14) ou destiné à saisir une rugosité de surface d'une surface d'un corps testé (14), ledit système étant pourvu
- d'un dispositif de mesure (12) selon l'une quelconque des revendications 1 à 11,
- d'une table de mesure (25) destinée à recevoir le corps à tester (14),
- d'un dispositif de manipulation (17) destiné à faire passer un dispositif de mesure (12) d'une position initiale (21) à une position de mesure (22),
- d'un corps de base (16) sur lequel sont prévus au moins la table de mesure (25) et le dispositif de manipulation (17),
- d'une commande (33) destinée à activer et à effectuer, grâce au dispositif de mesure (12), une mesure sur le corps testé (14), laquelle active, grâce au dispositif de manipulation (17), au moins une pose d'un corps de pénétration (41) du dispositif de mesure (12) sur le corps testé (14), le mouvement de pénétration du corps de pénétration (41) dans la surface du corps testé (14) ou le mouvement de balayage du corps de pénétration (41) sur la surface du corps testé (14) étant activé par le dispositif de mesure (12).

13. Système de mesure selon la revendication 12, **caractérisé en ce que** le dispositif de saisie optique (29) est disposé sur le corps de base (16) de manière contiguë au dispositif de mesure (12), la table de mesure (25) pouvant être déplacée entre le dispositif de mesure (12) et le dispositif de saisie optique (29), ou le dispositif de mesure (12) et le dispositif de saisie optique (29) pouvant être déplacés par rapport à la table de mesure (25), et de préférence un mouvement de déplacement de la table de mesure (25), en particulier le long d'un axe situé dans le plan de la surface du corps testé (14), étant activé par la commande (33).

14. Procédé destiné à saisir des signaux de mesure pendant un mouvement de pénétration d'un corps de pénétration (41) dans une surface d'un corps testé (14) d'un dispositif de mesure (12) ou pendant un mouvement de balayage d'un corps de pénétration (41) sur une surface d'un corps testé (14), lors duquel le corps testé (14) est positionné sur une table de mesure (25) et le dispositif de mesure (12) est posé, dans une position de départ, sur le corps testé (14), lors duquel le mouvement de pénétration ou le mouvement de balayage du corps de pénétration (41) est activé grâce au dispositif générateur de force (44) qui comprend un dispositif d'entraînement (45) et un dispositif de transmission magnétique (45), le dispositif de transmission magnétique (66) comprenant un premier pôle magnétique et un deuxième pôle magnétique (67, 68) qui sont disposés entre eux avec un espacement donné et dont les pôles identiques sont orientés l'un vers l'autre, lors duquel le mouvement de pénétration du corps de pénétration (41) dans le corps testé (14) est activé par un mouvement d'avance du dispositif d'entraînement (43) effectué au moyen de la force magnétique ou lors duquel le mouvement de balayage du corps de pénétration (41) sur le corps testé (14) est activé par une force magnétique.

15. Procédé selon la revendication 14, **caractérisé en ce que**, en vue de mesurer la dureté de la surface du corps testé (14), le dispositif de mesure (12) est, dans une première étape, déplacé de manière à se rapprocher du corps testé (14), que le mouvement d'avance est mis à l'arrêt lorsque le dispositif de mesure (12) se pose sur le corps testé (14), qu'un mouvement de déplacement du corps de pénétration (41) est activé jusqu'à ce que celui-ci repose sur la surface du corps testé (14) et que cette positon est transmise à la commande (33) en tant que position zéro pour la mesure de dureté qui suit, ou **en ce que**, en vue de mesurer la résistance aux rayures de la surface du corps testé (14), le corps de pénétration (41) est soumis, dans une première étape, à un mouvement de déplacement avant que le dispositif de mesure (12) se pose sur la surface du corps testé (14) de sorte que le corps de pénétration (41) dépasse librement d'une face inférieure du boîtier (47), que le dispositif de mesure (12) est avancé vers le corps testé (14) et est mis à l'arrêt lorsque le corps de pénétration (41) se pose sur le corps testé (14) et que cette position est transmise à la commande (33) en tant que position zéro pour la mesure de résistance aux rayures qui suit, et **en ce que** pendant le mouvement de pénétration du corps de pénétration (41) dans le corps testé (14) qui est activé par la force magnétique, la table de mesure (25) est déplacée dans une direction perpendiculaire au mouvement de pénétration du corps de pénétration (41) et qu'une rayure est réalisée dans la surface du corps testé (14) et **en ce que** les signaux de mesure du premier organe de mesure (78) servant à mesurer la profondeur de pénétration et les signaux de mesure d'au moins un autre, à savoir un deuxième, organe de mesure (91) affecté au corps de pénétration (41) et servant à mesurer une déviation du corps de pénétration (41) le long du sens de déplacement du corps testé (14) ainsi que la force calculée à partir du mouvement d'avance de l'élément d'entraînement (96) saisi par le troisième organe de mesure (105) ou les signaux de mesure saisis par le quatrième organe de mesure (110) sont saisis et évalués.

16. Procédé selon l'une quelconque des revendications 14 à 15, **caractérisé en ce que** le dispositif générateur de force (44) est soumis à une force de test et qu'un mouvement de pénétration du corps de pénétration (41) dans la surface du corps testé (14) est saisi au moins par un premier organe de mesure (78), et de préférence que la force provenant du mouvement d'avance qui agit sur le corps de pénétration (41) et qui est saisie par un troisième organe de mesure (105) est calculée ou est saisie par un quatrième organe de mesure (110), et **en ce que** la profondeur de pénétration du corps de pénétration (41) dans le corps testé (14) est saisie par le premier organe de mesure (78) et **en ce que** la dureté de la surface du corps testé (14) est déterminée en fonction de la géométrie du corps de pénétration (41), à partir de la force de pénétration calculée ou saisie par le troisième ou le quatrième organe de mesure (105, 110) et à partir de la profondeur de pénétration saisie par le premier organe de mesure (78).

17. Procédé selon l'une quelconque des revendications 14 à 16, **caractérisé en ce que**, avant la réalisation de la rayure dans le corps testé (14), le dispositif de mesure (12) est posé sur la surface du corps testé (14) et est déplacé dans une direction perpendiculaire au mouvement de pose sur le corps testé (14) et que les signaux de mesure saisis par le premier organe de mesure (78) sont saisis et sont stockés en tant que données de profil avant rayure et/ou **en ce que**, après la réalisation de la rayure dans le corps testé (14), le dispositif de mesure (12) est posé dans la rayure et que le corps de pénétration comportant le dispositif de mesure (12) est déplacé dans une direction perpendiculaire au mouvement de pose sur le corps testé (14) et que les signaux saisis par le dispositif de mesure (12) dans la rayure, le long du mouvement de déplacement du corps de pénétration (41), sont saisis et stockés en tant que données de profil après rayure.
